# EUROPEAN PATENT APPLICATION

(11) **EP 4 495 106 A1**
(43) Date of publication of application: **22.01.2025**
(21) Application number: 23771025.6
(22) Date of filing: 09.03.2023
(51) Int. Cl.: C07D 285/12, A61K 31/433, A61P 29/00, A23L 33/10

(54) **THIADIAZOLE COMPOUND, AND PHARMACEUTICAL COMPOSITION FOR PREVENTING OR TREATING INFLAMMATORY DISEASES, INCLUDING SAME**

(30) Priority: 16.03.2022 KR 20220032759
(71) Applicant: Daegu-Gyeongbuk Medical Innovation Foundation, Daegu 41061 (KR)
(72) Inventor: CHIN, Jungwook, Daegu 41061 (KR); KADAYAT, Tara Man, Daegu 41061 (KR); CHO, Sung Jin, Daegu 41061 (KR); JEON, Yong Hyun, Daegu 41061 (KR); KIM, Jina, Daegu 41061 (KR); KWON, Sugyeong, Daegu 41061 (KR); HWANG, Hayoung, Daegu 41061 (KR); LEE, Jae-Eon, Daegu 41061 (KR); KIM, Nayeon, Daegu 41061 (KR); JUNG, Kyungjin, Daegu 41061 (KR); KIM, Shinae, Daegu 41061 (KR); HWANG, Ji Sun, Daegu 41061 (KR)
(74) Representative: Mewburn Ellis LLP
(86) International application number: PCT/KR2023/003210
(87) International publication number: WO 2023/177146

(57) **Abstract**

The present disclosure relates to: a novel thiadiazole derivative compound; and a pharmaceutical composition for preventing or treating inflammatory diseases, the composition including the novel thiadiazole derivative compound as an active ingredient. The compound according to one embodiment is an effective PPARδ agonist which is effective in inhibiting inflammatory factors and nitric oxide production, and is effective in preventing the infiltration of inflammatory sites by macrophages, and thus can be effectively used as a pharmaceutical composition for preventing or treating inflammatory diseases.

## Description

### Technical Field

The present disclosure relates to a thiadiazole compound, and a pharmaceutical composition for preventing or treating inflammatory disease including the same.

### Background Art

Peroxisome proliferator-activated receptors (PPARs) are members of the nuclear receptor ligand-activated transcription factors. According to their ligand specificity, physiological role, and tissue distribution, PPARs are classified into three types of PPARα, PPARγ, and PPARδ. In particular, PPARδ (also known as PPARβ) is one of the PPAR transcription factors essential for several biological functions, and unlike PPARα and PPARγ, PPARδ is ubiquitously expressed. Therefore, its function and application in the field of drug development are the least known among PPARs. However, since the discovery of high affinity PPARδ agonists such as GW0742 and GW501516, several researchers have studied the role of PPARδ in diseases such as obesity and diabetes, as well as cancer, neurological disorders, inflammation, dyslipidemia, heart disease, and liver disease.

### Disclosure of the Invention

### Technical Goals

One embodiment is intended to provide a novel thiadiazole derivative compound.

Another embodiment is intended to provide a pharmaceutical composition including a thiadiazole derivative compound for preventing or treating inflammatory disease.

Another embodiment is intended to provide a health functional food composition for preventing or ameliorating inflammatory disease including a thiadiazole derivative compound.

Another embodiment is intended to provide a method for treating inflammatory disease, including administering the compound represented by Chemical Formula 1, a stereoisomer thereof, a hydrate thereof, or a pharmaceutically acceptable salt thereof to an individual or subject in need thereof.

Another embodiment is intended to provide the compound represented by Chemical Formula 1, a stereoisomer thereof, a hydrate thereof, or a pharmaceutically acceptable salt thereof for use in a treatment of inflammatory disease.

Another embodiment is intended to provide a use of the compound, a stereoisomer thereof, a hydrate thereof, or a pharmaceutically acceptable salt thereof for use in the manufacture of a drug for treating inflammatory disease.

### Technical Solutions

One embodiment provides a compound represented by the following Chemical Formula 1, a stereoisomer thereof, a hydrate thereof, or a salt thereof.

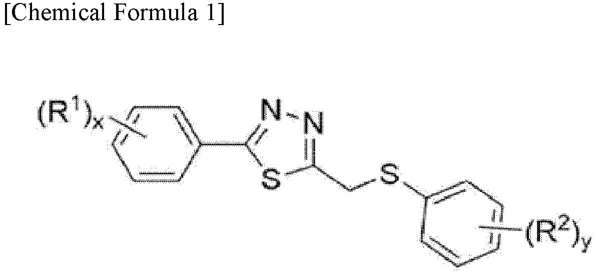

In the Chemical Formula 1,
R¹ is each independently -H, halogen, C₁₋₅alkyl, C₁₋₅haloalkyl, C₁₋₅alkoxy or C₁₋₅haloalkoxy;
R² is each independently -H, C₁₋₅alkyl or -O-R²¹,
wherein the R²¹ is cyano C₁₋₅alkyl, heteroaryl C₁₋₅alkyl of 5 to 8 atoms containing one or more heteroatoms selected from a group consisting of N, O and S, or 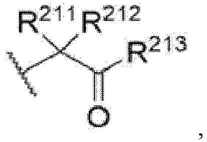
wherein the R²¹¹ and R²¹² are each independently -H or C₁₋₅alkyl, the R²¹³ is -OH, -NH₂, -NHOR²¹⁴ or -NHCN, and
the R²¹⁴ is -H or C₁₋₅alkyl; and
x and y are each independently integers from 1 to 5.

Another embodiment provides a pharmaceutical composition for preventing or treating inflammatory disease, including the compound represented by Chemical Formula 1, a stereoisomer thereof, a hydrate thereof, or a pharmaceutically acceptable salt thereof as an active ingredient.

Another embodiment provides a health functional food composition for preventing or ameliorating inflammatory disease, including the compound represented by Chemical Formula 1, a stereoisomer thereof, a hydrate thereof, or a pharmaceutically acceptable salt thereof as an active ingredient.

Another embodiment provides a method for treating inflammatory disease, including administering the compound represented by Chemical Formula 1, a stereoisomer thereof, a hydrate thereof, or a pharmaceutically acceptable salt thereof to an individual or subject in need thereof.

Another embodiment provides the compound represented by Chemical Formula 1, a stereoisomer thereof, a hydrate thereof, or a pharmaceutically acceptable salt thereof for use in the treatment of inflammatory disease.

*Another embodiment provides a use of the compound, a stereoisomer thereof, a hydrate thereof, or a pharmaceutically acceptable salt thereof for use in the manufacture of a drug for treating inflammatory disease.

### Advantageous Effects

The present disclosure relates to a novel thiadiazole derivative compound and a pharmaceutical composition for preventing or treating inflammatory disease including the same as an active ingredient, the compound according to one embodiment is an effective PPARδ agonist that can effectively inhibit the production of inflammatory factors and nitric oxide, and is effective in preventing the infiltration of macrophages into the inflammatory site, so it can be usefully used as a pharmaceutical composition for preventing or treating inflammatory disease.

### Brief Description of Drawings

FIG. 1 is a diagram showing results of analyzing cytotoxicity of a compound of Example 1.
FIG. 2 is a diagram showing results of analyzing a nitric oxide (NO) production inhibitory effect of a compound of Example 1.
FIG. 3 is a diagram showing results of analyzing an NO synthase iNOS protein expression inhibitory effect of a compound of Example 1.
FIG. 4 is a diagram showing results of analyzing an inflammatory factors TNF-α and IL-6 production inhibitory effect of a compound of Example 1.
FIG. 5 is a diagram showing results of analyzing an inflammatory factor NF-κ activity inhibitory effect of a compound of Example 1.
FIG. 6 is a diagram showing results of analyzing an inflammation-related p-IKKαβ protein expression reduction effect of a compound of Example 1.
FIG. 7 is a diagram showing a macrophage tracking experiment process conducted using a compound of Experimental Example 1.
FIG. 8 and FIG. 9 are diagrams showing results of analyzing a macrophage infiltration inhibitory effect of a compound of Experimental Example 1 using mice induced with acute inflammation.

### Best Mode for Carrying Out the Invention

The embodiments described herein may be modified into various other forms, and the technology according to one embodiment is not limited to the embodiments described below. In addition, the embodiment of one embodiment is provided to more completely explain the present disclosure to those with average knowledge in the relevant technical field. Furthermore, throughout the specification, "comprising" a certain element means that other elements may be further included rather than excluding other elements, unless specifically stated to the contrary.

As used herein, numerical ranges include lower and upper limits and all values within that range, increments that are logically derived from the shape and width of the range being defined, all double restricted values, and all possible combinations of upper and lower limits of numerical ranges defined in different forms. As an example, if the content of the composition is limited to 10% to 80% or 20% to 50%, the numerical range of 10% to 50% or 50% to 80% should also be interpreted as described herein. Unless otherwise specified herein, values outside the numerical range that may occur due to experimental error or rounding of values are also included in the defined numerical range.

Hereinafter, unless otherwise specified herein, "about" may be considered a value within 30%, 25%, 20%, 15%, 10% or 5% of the specified value.

One embodiment provides a compound represented by the following Chemical Formula 1, a stereoisomer thereof, a hydrate thereof, or a salt thereof.

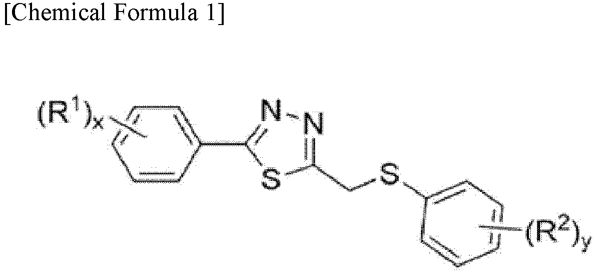

In the Chemical Formula 1,
R¹ is each independently -H, halogen, C₁₋₅alkyl, C₁₋₅haloalkyl, C₁₋₅alkoxy or C₁₋₅haloalkoxy;
R² is each independently -H, C₁₋₅alkyl or -O-R²¹,
wherein the R²¹ is cyano C₁₋₅alkyl, heteroaryl C₁₋₅alkyl of 5 to 8 atoms containing one or more heteroatoms selected from the group consisting of N, O and S, or 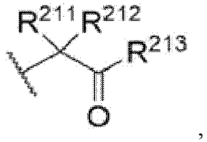
wherein the R²¹¹ and R²¹² are each independently -H or C₁₋₅alkyl, the R²¹³ is -OH, -NH₂, -NHOR²¹⁴ or -NHCN, and
the R²¹⁴ is -H or C₁₋₅alkyl; and
x and y are each independently integers from 1 to 5.

In one embodiment, the compound represented by the Chemical Formula 1 may be a compound represented by the following Chemical Formula 2.

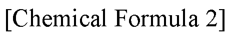

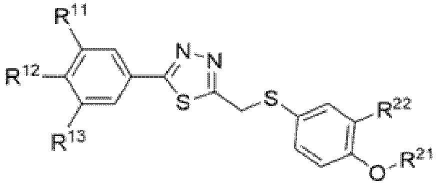

In the Chemical Formula 2,
R¹¹, R¹² and R¹³ are each independently -H, halogen, C₁₋₅alkyl, C₁₋₅haloalkyl or C₁₋₅haloalkoxy;
R²² is -H or C₁₋₅alkyl; and
R²¹ is cyano C₁₋₅alkyl, heteroaryl C₁₋₅alkyl of 5 to 8 atoms containing one or more heteroatoms selected from the group consisting of N, O and S, or 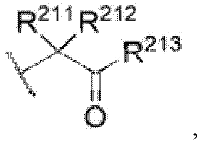
wherein the R²¹¹ and R²¹² are each independently -H or C₁₋₅alkyl, the R²¹³ is -OH, -NH₂, -NHOR²¹⁴ or -NHCN, and
the R²¹⁴ is -H or C₁₋₅alkyl.

In one embodiment, the compound represented by the Chemical Formula 1 may be a compound represented by the following Chemical Formula 3.

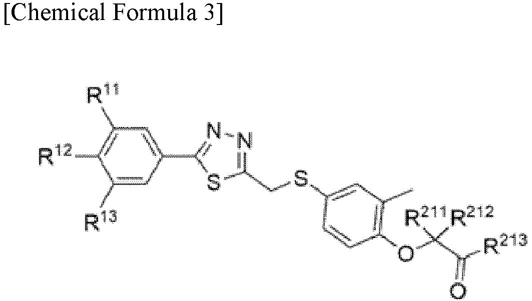

In the Chemical Formula 3,
R¹¹, R¹² and R¹³ are each independently -H, halogen, C₁₋₅alkyl, C₁₋₅haloalkyl or C₁₋₅haloalkoxy;
R²¹¹ and R²¹² are each independently -H or C₁₋₅alkyl; and
R²¹³ is -OH, -NH₂, -NHOR²¹⁴ or -NHCN, and the R²¹⁴ is -H or C₁₋₅alkyl.

In one embodiment, the R¹, R¹¹, R¹² and R¹³ may each independently be C₁₋₄alkyl, C₁₋₃alkyl, C₁₋₂alkyl, -CH₃, C₁₋₄haloalkyl, C₁₋₃haloalkyl, C₁₋₂haloalkyl, C₁haloalkyl, -CF₃, C₁₋₄haloalkoxy, C₁₋₃haloalkoxy, C₁₋₂haloalkoxy, C₁haloalkoxy or -OCF₃.

In one embodiment, the R² may each independently be C₁₋₄alkyl, C₁₋₃alkyl, C₁₋₂alkyl, -CH₃.

In one embodiment, the R²¹ may be cyano C₁₋₄alkyl, cyano C₁₋₃alkyl, cyano C₁₋₂alkyl, heteroaryl C₁₋₄alkyl of 5 to 6 atoms containing one or more heteroatoms selected from a group consisting of N, O and S, heteroaryl C₁₋₃alkyl of 5 to 6 atoms containing one or more heteroatoms selected from a group consisting of N, O and S, heteroaryl C₁₋₂alkyl of 5 to 6 atoms containing one or more heteroatoms selected from a group consisting of N, O and S, heteroaryl C₁alkyl of 5 to 6 atoms containing one or more heteroatoms selected from a group consisting of N, O and S,

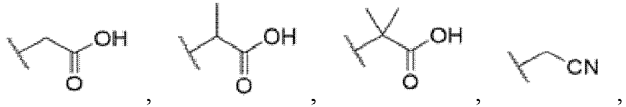

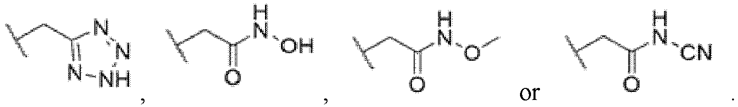

In one embodiment, the R²¹¹ and R²¹² may each independently be C₁₋₄alkyl, C₁₋₃alkyl, C₁₋₂alkyl, -CH₃.

In one embodiment, the R²¹⁴ may each independently be C₁₋₄alkyl, C₁₋₃alkyl, C₁₋₂alkyl, -CH₃.

In one embodiment, the x and y may each independently be integers of 1 to 4 or 1 to 3.

In one embodiment, the compound represented by Chemical Formula 1 may be any one selected from the following group of compounds.
(1) 2-(2-methyl-4-(((5-(4-(trifluoromethyl)phenyl)-1,3,4-thiadiazol-2-yl)methyl)thio)phenoxy)acetic acid;
(2) 2-(2-methyl-4-(((5-(4-(trifluoromethyl)phenyl)-1,3,4-thiadiazol-2-yl)methyl)thio)phenoxy)propanoic acid;
(3) 2-methyl-2-(2-methyl-4-(((5-(4-(trifluoromethyl)phenyl)-1,3,4-thiadiazol-2-yl)methyl)thio)phenoxy)propanoic acid;
(4) 2-(2-methyl-4-(((5-(4-(trifluoromethyl)phenyl)-1,3,4-thiadiazol-2-yl)methyl)thio)phenoxy)acetonitrile;
(5) 2-(((4-((2H-tetrazol-5-yl)methoxy)-3-methylphenyl)thio)methyl)-5-(4-(trifluoromethyl)phenyl)-1,3,4-thiadiazole;
(6) N-hydroxy-2-(2-methyl-4-(((5-(4-(trifluoromethyl)phenyl)-1,3,4-thiadiazol-2-yl)methyl)thio)phenoxy)acetamide;
(7) N-methoxy-2-(2-methyl-4-(((5-(4-(trifluoromethyl)phenyl)-1,3,4-thiadiazol-2-yl)methyl)thio)phenoxy)acetamide;
(8) N-cyano-2-(2-methyl-4-(((5-(4-(trifluoromethyl)phenyl)-1,3,4-thiadiazol-2-yl)methyl)thio)phenoxy)acetamide;
(9) 2-(4-(((5-(4-fluorophenyl)-1,3,4-thiadiazol-2-yl)methyl)thio)-2-methylphenoxy)acetic acid;
(10) 2-(4-(((5-(4-fluorophenyl)-1,3,4-thiadiazol-2-yl)methyl)thio)-2-methylphenoxy)propanoic acid;
(11) 2-(4-(((5-(4-fluorophenyl)-1,3,4-thiadiazol-2-yl)methyl)thio)-2-methylphenoxy)-2-methylpropanoic acid;
(12) 2-(4-(((5-(3,4-difluorophenyl)-1,3,4-thiadiazol-2-yl)methyl)thio)-2-methylphenoxy)acetic acid;
(13) 2-(4-(((5-(3,4-difluorophenyl)-1,3,4-thiadiazol-2-yl)methyl)thio)-2-methylphenoxy)propanoic acid;
(14) 2-(4-(((5-(3,4-difluorophenyl)-1,3,4-thiadiazol-2-yl)methyl)thio)-2-methylphenoxy)-2-methylpropanoic acid;
(15) 2-(2-methyl-4-(((5-(3,4,5-trifluorophenyl)-1,3,4-thiadiazol-2-yl)methyl)thio)phenoxy)acetic acid;
(16) 2-(4-(((5-(3-chloro-4-fluorophenyl)-1,3,4-thiadiazol-2-yl)methyl)thio)-2-methylphenoxy)acetic acid;
(17) 2-(4-(((5-(3-chloro-4-fluorophenyl)-1,3,4-thiadiazol-2-yl)methyl)thio)-2-methylphenoxy)propanoic acid;
(18) 2-(4-(((5-(3-chloro-4-fluorophenyl)-1,3,4-thiadiazol-2-yl)methyl)thio)-2-methylphenoxy)-2-methylpropanoic acid;
(19) 2-(4-(((5-(3-fluoro-4-(trifluoromethyl)phenyl)-1,3,4-thiadiazol-2-yl)methyl)thio)-2-methylphenoxy)acetic acid;
(20) 2-(4-(((5-(3-fluoro-4-(trifluoromethyl)phenyl)-1,3,4-thiadiazol-2-yl)methyl)thio)-2-methylphenoxy)propanoic acid;
(21) 2-(4-(((5-(3-fluoro-4-(trifluoromethyl)phenyl)-1,3,4-thiadiazol-2-yl)methyl)thio)-2-methylphenoxy)-2-methylpropanoic acid;
(22) 2-(2-methyl-4-(((5-(p-tolyl)-1,3,4-thiadiazol-2-yl)methyl)thio)phenoxy)acetic acid;
(23) 2-(2-methyl-4-(((5-(p-tolyl)-1,3,4-thiadiazol-2-yl)methyl)thio)phenoxy)propanoic acid;
(24) 2-methyl-2-(2-methyl-4-(((5-(p-tolyl)-1,3,4-thiadiazol-2-yl)methyl)thio)phenoxy)propanoic acid;
(25) 2-(2-methyl-4-(((5-(4-(trifluoromethoxy)phenyl)-1,3,4-thiadiazol-2-yl)methyl)thio)phenoxy)acetic acid;
(26) 2-(2-methyl-4-(((5-(4-(trifluoromethoxy)phenyl)-1,3,4-thiadiazol-2-yl)methyl)thio)phenoxy)propanoic acid; and
(27) 2-methyl-2-(2-methyl-4-(((5-(4-(trifluoromethoxy)phenyl)-1,3,4-thiadiazol-2-yl)methyl)thio)phenoxy)propanoic acid.

In one embodiment, the compound represented by Chemical Formula 1 can be used in the form of a pharmaceutically acceptable salt, and an acid addition salt formed by a pharmaceutically acceptable free acid is useful as the salt. Acid addition salts are obtained from inorganic acids such as hydrochloric acid, nitric acid, phosphoric acid, sulfuric acid, hydrobromic acid, hydroiodic acid, nitrous acid, phosphorous acid, etc., non-toxic organic acids such as aliphatic mono and dicarboxylates, phenyl-substituted alkanoates, hydroxy alkanoates and alkanedioates, aromatic acids, aliphatic and aromatic sulfonic acids, etc., organic acids such as trifluoroacetic acid, acetate, benzoic acid, citric acid, lactic acid, maleic acid, gluconic acid, methanesulfonic acid, 4-toluenesulfonic acid, tartaric acid, fumaric acid, etc. These types of pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxybenzoate, phthalate, terephthalate, benzenesulfonate, toluenesulfonate, chlorobenzenesulfonate, xylene sulfonate, phenylacetate, phenylpropionate, phenylbutyrate, citrate, lactate, β-hydroxybutyrate, glycolate, malate, tartrate, methanesulfonate, propane sulfonate, naphthalene-1-sulfonate, naphthalene-2-sulfonate, mandelate etc.

One embodiment includes not only the compound represented by Chemical Formula 1 and a pharmaceutically acceptable salt thereof, but also solvates, optical isomers, hydrates, etc., that can be prepared therefrom.

One embodiment provides a pharmaceutical composition for preventing or treating inflammatory disease, including a compound represented by the following Chemical Formula 1, a stereoisomer thereof, a hydrate thereof, or a pharmaceutically acceptable salt thereof as an active ingredient.

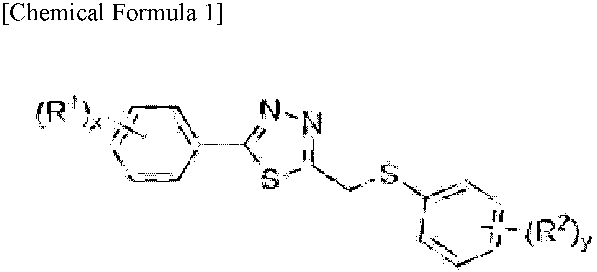

In the Chemical Formula 1,
R¹ is each independently -H, halogen, C₁₋₅alkyl, C₁₋₅haloalkyl, C₁₋₅alkoxy or C₁₋₅haloalkoxy;
R² is each independently -H, C₁₋₅alkyl or -O-R²¹,
wherein the R²¹ is cyano C₁₋₅alkyl, heteroaryl C₁₋₅alkyl of 5 to 8 atoms containing one or more heteroatoms selected from the group consisting of N, O and S, or 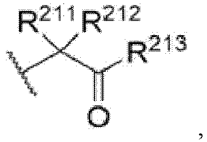
wherein the R²¹¹ and R²¹² are each independently -H or C₁₋₅alkyl, the R²¹³ is - OH, -NH₂, -NHOR²¹⁴ or -NHCN, and
the R²¹⁴ is -H or C₁₋₅alkyl; and
x and y are each independently integers from 1 to 5.

In one embodiment, the compound represented by the Chemical Formula 1 may be a compound represented by the following Chemical Formula 2.

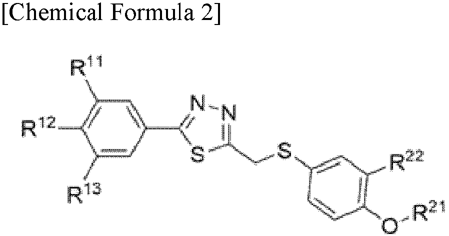

In the Chemical Formula 2,
R¹¹, R¹² and R¹³ are each independently -H, halogen, C₁₋₅alkyl, C₁₋₅haloalkyl or C₁₋₅haloalkoxy;
R²² is -H or C₁₋₅alkyl; and
R²¹ is cyano C₁₋₅alkyl, heteroaryl C₁₋₅alkyl of 5 to 8 atoms containing one or more heteroatoms selected from the group consisting of N, O and S, or 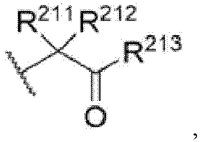
wherein the R²¹¹ and R²¹² are each independently -H or C₁₋₅alkyl, the R²¹³ is - OH, -NH₂, -NHOR²¹⁴ or -NHCN, and
the R²¹⁴ is -H or C₁₋₅alkyl.

In one embodiment, the compound represented by the Chemical Formula 1 may be a compound represented by the following Chemical Formula 3.

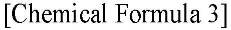

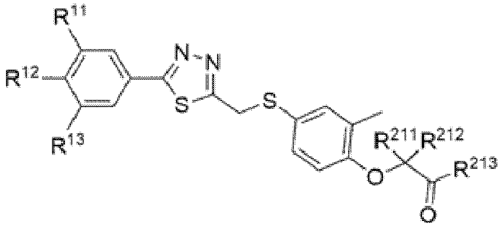

In the Chemical Formula 3,
R¹¹, R¹² and R¹³ are each independently -H, halogen, C₁₋₅alkyl, C₁₋₅haloalkyl or C₁₋₅haloalkoxy;
R²¹¹ and R²¹² are each independently -H or C₁₋₅alkyl; and
R²¹³ is -OH, -NH₂, -NHOR²¹⁴ or -NHCN, and the R²¹⁴ is -H or C₁₋₅alkyl.

In one embodiment, the R¹, R¹¹, R¹² and R¹³ may each independently be C₁₋₄alkyl, C₁₋₃alkyl, C₁₋₂alkyl, -CH₃, C₁₋₄haloalkyl, C₁₋₃haloalkyl, C₁₋₂haloalkyl, C₁haloalkyl, - CF₃, C₁₋₄haloalkoxy, C₁₋₃haloalkoxy, C₁₋₂haloalkoxy, C₁haloalkoxy or -OCF₃.

In one embodiment, the R² may each independently be C₁₋₄alkyl, C₁₋₃alkyl, C₁₋₂alkyl, -CH₃.

In one embodiment, the R²¹ may be cyano C₁₋₄alkyl, cyano C₁₋₃alkyl, cyano C₁₋₂alkyl, heteroaryl C₁₋₄alkyl of 5 to 6 atoms containing one or more heteroatoms selected from a group consisting of N, O and S, heteroaryl C₁₋₃alkyl of 5 to 6 atoms containing one or more heteroatoms selected from a group consisting of N, O and S, heteroaryl C₁₋₂alkyl of 5 to 6 atoms containing one or more heteroatoms selected from a group consisting of N, O and S, heteroaryl C₁alkyl of 5 to 6 atoms containing one or more heteroatoms selected from a group consisting of N, O and S,

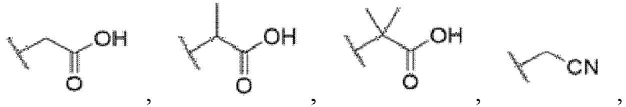

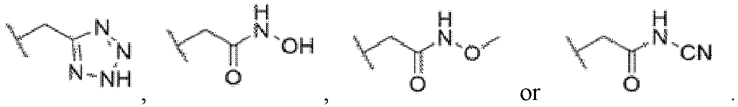

In one embodiment, the R²¹¹ and R²¹² may each independently be C₁₋₄alkyl, C₁₋₃alkyl, C₁₋₂alkyl, -CH₃.

In one embodiment, the R²¹⁴ may each independently be C₁₋₄alkyl, C₁₋₃alkyl, C₁₋₂alkyl, -CH₃.

In one embodiment, the x and y may each independently be integers of 1 to 4 or 1 to 3.

In one embodiment, the compound represented by Chemical Formula 1 may be any one selected from the following group of compounds.
(1) 2-(2-methyl-4-(((5-(4-(trifluoromethyl)phenyl)-1,3,4-thiadiazol-2-yl)methyl)thio)phenoxy)acetic acid;
(2) 2-(2-methyl-4-(((5-(4-(trifluoromethyl)phenyl)-1,3,4-thiadiazol-2-yl)methyl)thio)phenoxy)propanoic acid;
(3) 2-methyl-2-(2-methyl-4-(((5-(4-(trifluoromethyl)phenyl)-1,3,4-thiadiazol-2-yl)methyl)thio)phenoxy)propanoic acid;
(4) 2-(2-methyl-4-(((5-(4-(trifluoromethyl)phenyl)-1,3,4-thiadiazol-2-yl)methyl)thio)phenoxy)acetonitrile;
(5) 2-(((4-((2H-tetrazol-5-yl)methoxy)-3-methylphenyl)thio)methyl)-5-(4-(trifluoromethyl)phenyl)-1,3,4-thiadiazole;
(6) N-hydroxy-2-(2-methyl-4-(((5-(4-(trifluoromethyl)phenyl)-1,3,4-thiadiazol-2-yl)methyl)thio)phenoxy)acetamide;
(7) N-methoxy-2-(2-methyl-4-(((5-(4-(trifluoromethyl)phenyl)-1,3,4-thiadiazol-2-yl)methyl)thio)phenoxy)acetamide;
(8) N-cyano-2-(2-methyl-4-(((5-(4-(trifluoromethyl)phenyl)-1,3,4-thiadiazol-2-yl)methyl)thio)phenoxy)acetamide;
(9) 2-(4-(((5-(4-fluorophenyl)-1,3,4-thiadiazol-2-yl)methyl)thio)-2-methylphenoxy)acetic acid;
(10) 2-(4-(((5-(4-fluorophenyl)-1,3,4-thiadiazol-2-yl)methyl)thio)-2-methylphenoxy)propanoic acid;
(11) 2-(4-(((5-(4-fluorophenyl)-1,3,4-thiadiazol-2-yl)methyl)thio)-2-methylphenoxy)-2-methylpropanoic acid;
(12) 2-(4-(((5-(3,4-difluorophenyl)-1,3,4-thiadiazol-2-yl)methyl)thio)-2-methylphenoxy)acetic acid;
(13) 2-(4-(((5-(3,4-difluorophenyl)-1,3,4-thiadiazol-2-yl)methyl)thio)-2-methylphenoxy)propanoic acid;
(14) 2-(4-(((5-(3,4-difluorophenyl)-1,3,4-thiadiazol-2-yl)methyl)thio)-2-methylphenoxy)-2-methylpropanoic acid;
(15) 2-(2-methyl-4-(((5-(3,4,5-trifluorophenyl)-1,3,4-thiadiazol-2-yl)methyl)thio)phenoxy)acetic acid;
(16) 2-(4-(((5-(3-chloro-4-fluorophenyl)-1,3,4-thiadiazol-2-yl)methyl)thio)-2-methylphenoxy)acetic acid;
(17) 2-(4-(((5-(3-chloro-4-fluorophenyl)-1,3,4-thiadiazol-2-yl)methyl)thio)-2-methylphenoxy)propanoic acid;
*(18) 2-(4-(((5-(3-chloro-4-fluorophenyl)-1,3,4-thiadiazol-2-yl)methyl)thio)-2-methylphenoxy)-2-methylpropanoic acid;
(19) 2-(4-(((5-(3-fluoro-4-(trifluoromethyl)phenyl)-1,3,4-thiadiazol-2-yl)methyl)thio)-2-methylphenoxy)acetic acid;
(20) 2-(4-(((5-(3-fluoro-4-(trifluoromethyl)phenyl)-1,3,4-thiadiazol-2-yl)methyl)thio)-2-methylphenoxy)propanoic acid;
(21) 2-(4-(((5-(3-fluoro-4-(trifluoromethyl)phenyl)-1,3,4-thiadiazol-2-yl)methyl)thio)-2-methylphenoxy)-2-methylpropanoic acid;
(22) 2-(2-methyl-4-(((5-(p-tolyl)-1,3,4-thiadiazol-2-yl)methyl)thio)phenoxy)acetic acid;
*(23) 2-(2-methyl-4-(((5-(p-tolyl)-1,3,4-thiadiazol-2-yl)methyl)thio)phenoxy)propanoic acid;
(24) 2-methyl-2-(2-methyl-4-(((5-(p-tolyl)-1,3,4-thiadiazol-2-yl)methyl)thio)phenoxy)propanoic acid;
(25) 2-(2-methyl-4-(((5-(4-(trifluoromethoxy)phenyl)-1,3,4-thiadiazol-2-yl)methyl)thio)phenoxy)acetic acid;
(26) 2-(2-methyl-4-(((5-(4-(trifluoromethoxy)phenyl)-1,3,4-thiadiazol-2-yl)methyl)thio)phenoxy)propanoic acid; and
(27) 2-methyl-2-(2-methyl-4-(((5-(4-(trifluoromethoxy)phenyl)-1,3,4-thiadiazol-2-yl)methyl)thio)phenoxy)propanoic acid.

In one embodiment, the inflammatory disease is not particularly limited as long as it is an inflammatory disease. Examples of inflammatory diseases include, but are not limited to, inflammatory bowel disease, osteoarthritis, bronchitis, rheumatoid arthritis, degenerative arthritis, asthma, atopy, diabetes, myocardial infarction, Crohn's disease, or psoriasis.

The compound represented by Chemical Formula 1 or a pharmaceutically acceptable salt thereof may be administered in various oral and parenteral formulations during clinical administration. In the case of formulation, it is prepared using diluents or excipients such as fillers, extenders, binders, wetting agents, disintegrants, and surfactants that are conventionally used. Solid preparations for oral administration include tablets, pills, acids, granules, and capsules, etc., and such solid preparations may be prepared by mixing, in addition to one or more compounds, at least one or more excipients such as starch, calcium carbonate, sucrose, lactose, and gelatin. In addition to simple excipients, lubricants such as magnesium stearate and talc are also used. Liquid preparations for oral administration include suspensions, oral solutions, emulsions, syrups, etc., and in addition to the commonly used simple diluents such as water and liquid paraffin, various excipients such as wetting agents, sweeteners, aromatics, and preservatives, etc., may be included. Preparations for parenteral administration include sterilized aqueous solutions, non-aqueous solvents, suspensions, and emulsions. As non-aqueous solvents and suspensions, propylene glycol, polyethylene glycol, vegetable oils such as olive oil, and injectable ester such as ethyl oleate may be used.

A pharmaceutical composition including the compound represented by Chemical Formula 1 or a pharmaceutically acceptable salt thereof as an active ingredient can be administered parenterally, and parenteral administration is by subcutaneous injection, intravenous injection, intramuscular injection, or intrathoracic injection.

At this time, in order to formulate into a formulation for parenteral administration, the compound represented by Chemical Formula 1 or a pharmaceutically acceptable salt thereof is mixed with water along with a stabilizer or buffer to prepare a solution or suspension, which can be prepared in an ampoule or vial unit dosage form. The composition may be sterile and/or contain auxiliaries such as preservatives, stabilizers, wetting agents or emulsification accelerators, salts and/or buffers for adjusting osmotic pressure, and other therapeutically useful substances, and may be formulated according to conventional mixing, granulating or coating methods.

The formulations for oral administration include, for example, tablets, pills, hard/soft capsules, solutions, suspensions, emulsifiers, syrups, granules, elixirs, troches, etc., and in addition to the active ingredients, such formulations contain diluents (e.g., lactose, dextrose, sucrose, mannitol, sorbitol, cellulose and/or glycine) and lubricants (e.g., silica, talc, stearic acid and magnesium or calcium salts thereof and/or polyethylene glycol). The tablets may contain binders such as magnesium aluminum silicate, starch paste, gelatin, methylcellulose, sodium carboxymethylcellulose and/or polyvinylpyrrolidine, etc., and depending on cases, may contain disintegrants such as starch, agar, alginic acid or sodium salts thereof, etc., or effervescent mixtures and/or absorbents, colorants, flavoring agents, and sweeteners.

One embodiment provides a health functional food composition for preventing or ameliorating inflammatory disease, including the compound represented by Chemical Formula 1, a stereoisomer thereof, a hydrate thereof, or a salt thereof as an active ingredient.

The compound represented by Chemical Formula 1 according to one embodiment may be added to food as is or used together with other foods or food ingredients, and may be used appropriately according to conventional methods. The mixing amount of the active ingredient may be appropriately determined depending on the purpose of use (prevention or amelioration). Generally, the amount of the above compound in health food may be 0.1 to 90 parts by weight of the total weight of the food. However, in the case of long-term intake for health and hygiene purposes or health control, the amount may be below the above range, and since there is no problem in terms of safety, the active ingredient may be used in amounts exceeding the above range.

In addition, the health functional food composition according to one embodiment has no particular restrictions on other ingredients other than containing the above compound, and may contain various flavoring agents or natural carbohydrates as additional ingredients like ordinary beverages.

Furthermore, it may contain various nutrients, vitamins, minerals (electrolytes), flavoring agents such as synthetic flavoring agents and natural flavoring agents, colorants and fillers (cheese, chocolate, etc.), pectic acid and salts thereof, alginic acid and salts thereof, organic acids, protective colloidal thickeners, pH adjusters, stabilizers, preservatives, glycerin, alcohol, carbonating agents used in carbonated beverages, etc.

One embodiment provides a method for treating inflammatory disease, including administering the compound represented by Chemical Formula 1, a stereoisomer thereof, a hydrate thereof, or a pharmaceutically acceptable salt thereof to an individual or subject in need thereof.

One embodiment provides the compound represented by Chemical Formula 1, a stereoisomer thereof, a hydrate thereof, or a pharmaceutically acceptable salt thereof for use in the treatment of inflammatory disease.

One embodiment provides a use of the compound, a stereoisomer thereof, a hydrate thereof, or a pharmaceutically acceptable salt thereof for use in the manufacture of a drug for treating inflammatory disease.

### Modes for Carrying Out the Invention

Hereinafter, examples and experimental examples will be described in detail below. However, the examples and experimental examples described below are only illustrative of some, and the technology described in this specification is not limited thereto.

### <Example 1> Preparation of 2-(2-methyl-4-(((5-(4-(trifluoromethyl)phenyl)-1,3,4-thiadiazol-2-yl)methyl)thio)phenoxy)acetic acid

### Step 1: Preparation of 4-(trifluoromethyl)benzohydrazide (b-1)

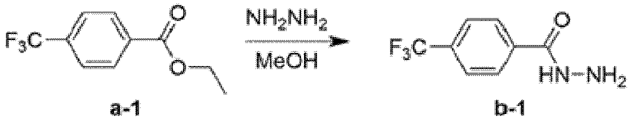

Ethyl 4-(trifluoromethyl)benzoate (2.033 mL, 11.46 mmol) was dissolved in MeOH (10 mL), hydrazine hydrate (1.671 mL, 27.5 mmol) was added at 0°C and stirred for 5 hours at 25°C, followed by stirring at 70°C for 1 hour. After the reaction was completed, cold water was added to solidify to obtain compound b-1 (2.21 g, 10.83 mmol).

### Step 2: Preparation of ethyl 2-oxo-2-(2-(4-(trifluoromethyl)benzoyl)hydrazinyl)acetate (c-1)

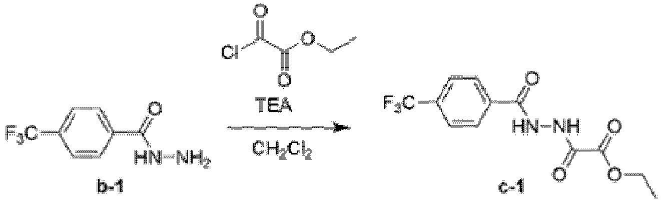

Compound b-1 (2.21 g, 10.83 mmol) was dissolved in dichloromethane (20 mL), TEA (1.521 mL, 10.83 mmol) was added dropwise at 0°C, and then ethyl 2-chloro-2-oxoacetate (1.210 mL, 10.83 mmol) was slowly added. The mixture was stirred at room temperature for 1 hour. After the reaction was completed, it was quenched with water, extracted with dichloromethane, dried over anhydrous Na₂SO₄, and concentrated under reduced pressure to obtain Compound c-1 (2.8 g, 85% yield). It is used without further purification in the next step.

### Step 3: Preparation of ethyl 5-(3,4,5-trifluorophenyl)-1,3,4-thiadiazole-2-carboxylate (d-1)

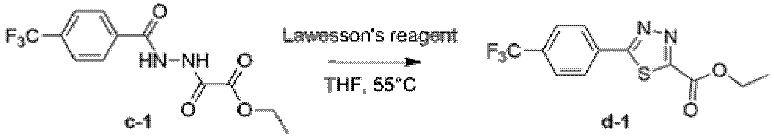

Compound c-1 (1 g, 3.29 mmol) was dissolved in THF (1 mL), Lawesson's Reagent (1.99 g, 4.93 mmol) was added, and the mixture was heated at 55°C for 12 hours. After the reaction was completed, it was filtered through celite, washed with NaHCO₃, and the organic layer was dried over anhydrous MgSO₄. The obtained residue was concentrated under reduced pressure and purified by MPLC (silica gel, 0-25%, EtOAc/Hexane) to obtain Compound d-1 (0.88 g, 89% yield).

ESI LC/MS: m/z calcd. for C₁₂H₁₀F₃N₂O₂S [M+H]⁺: 303.04; found 303.1. ¹H NMR (CD₃OD, 400MHz) δ8.31 (d, *J =* 7.2 Hz, 2H), 7.91 (d, *J =* 7.44 Hz, 2H), 4.55 (q, *J* = 6.64 Hz, 2H), 1.47 (t, *J =* 6.1 Hz, 3H).

### Step 4: Preparation of 5-(4-(trifluoromethyl)phenyl)-1,3,4-thiadiazol-2-yl)methanol (e-1)

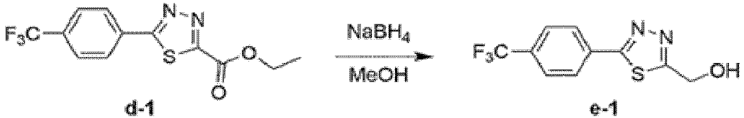

Compound d-1 (0.8 g, 2.64 mmol) was dissolved in methanol, and sodium borohydride (0.31 g, 7.94 mmol) was slowly added at 0°C, followed by stirring at room temperature for 1 hour. After the reaction is completed, it is quenched with NH₄Cl aqueous solution, concentrated under reduced pressure, extracted with ethyl acetate, and dried over anhydrous Na₂SO₄. The obtained residue was concentrated under reduced pressure, purified by MPLC (silica gel, 5-30%, EtOAc/Hexane), and concentrated under reduced pressure to obtain Compound e-1 (0.61 g, 89% yield) as a light yellow solid.

ESI LC/MS: m/z calcd. for C₁₀H₈F₃N₂OS [M+H]⁺: 261.03; found 261.11. ¹H NMR (CD₃OD, 400MHz) δ8.19 (d, *J =* 8.12 Hz, 2H), 7.86 (d, *J =* 8.24 Hz, 2H), 5.03 (s, 2H). ¹³C NMR (CD₃OD, 100MHz) 174.77, 167.79, 133.38, 132.17, 128.06, 127.63, 126.03, 125.91, 122.49, 58.61.

### Step 5: Preparation of 2-(chloromethyl)-5-(4-(trifluoromethyl)phenyl)-1,3,4-thiadiazole (f-1)

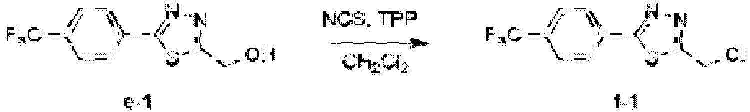

Compound e-1 (1 g, 3.84 mmol) was dissolved in CH₂Cl₂ (10 mL), then triphenylporphine (1.2 g, 4.61 mmol) and iothiocyanate (NCS) (0.616 g, 4.61 mmol) were added, followed by stirring at room temperature for 2 hours. After the reaction was completed, it was washed with water and saline solution and dried over anhydrous MgSO₄. Volatile substances were removed by concentration under reduced pressure, and the obtained residue was purified by MPLC (silica gel, 0-20%, EtOAc/Hexane) to obtain Compound f-1 (0.203 g, 19% yield).

ESI LC/MS: m/z calcd. for C₁₀H₇ClF₃N₂S [M+H]⁺: 279.01; found 279.1. ¹H NMR (CD₃OD, 400MHz) δ8.22 (d, *J =* 8.12 Hz, 2H), 7.87 (d, *J =* 8.24 Hz, 2H), 5.17 (s, 2H). ¹³C NMR (CD₃OD, 100MHz) δ169.38, 168.29, 133.05, 132.48, 128.20, 128.06, 125.98, 125.14, 122.45, 37.42.

### Step 6: Preparation of 2-methyl-4-(((5-(4-(trifluoromethyl)phenyl)-1,3,4-thiadiazol-2-yl)methyl)thio)phenol (g-1)

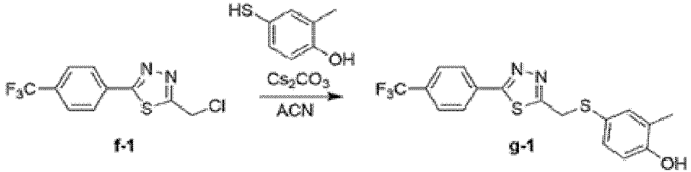

4-mercapto-2-methylphenol (0.27 g, 1.97 mmol) was dissolved in acetonitrile (8 mL), then cesium carbonate (0.64 g, 1.97 mmol) and Compound f-1 (0.5 g, 1.97 mmol) were added dropwise, followed by stirring at room temperature for 2 hours. After concentrating under reduced pressure to remove the solvent, it was extracted with ethyl acetate and dried over anhydrous Na₂SO₄. The obtained residue was concentrated under reduced pressure, purified by MPLC (silica gel, 5-20%, EtOAc/Hexane), and concentrated under reduced pressure to obtain Compound g-1 (0.64 g, 85% yield).

ESI LC/MS: m/z calcd. for C₁₇H₁₄F₃N₂OS₂ [M+H]⁺: 383.04; found 383.1. ¹H NMR (DMSO-d6, 400MHz) δ9.63 (s, 1H), 8.15 (d, *J =* 8.12 Hz, 2H), 7.91 (d, *J =* 8.28 Hz, 2H), 7.20 (s, 1H), 7.09 (d, *J =* 8.24 Hz, 1H), 6.71 (d, *J =* 8.28 Hz, 2H), 4.57 (s, 2H), 2.06 (s, 3H). ¹³C NMR (DMSO-d₆, 100MHz) δ171.03, 167.61, 156.28, 135.19, 133.72, 131.59, 128.85, 126.86, 126.83, 125.71, 121.19, 115.81, 34.02, 16.28.

### Step 7: Preparation of methyl 2-(2-methyl-4-(((5-(4-(trifluoromethyl)phenyl)-1,3,4-thiadiazol-2-yl)methyl)thio)phenoxy) acetate (h-1)

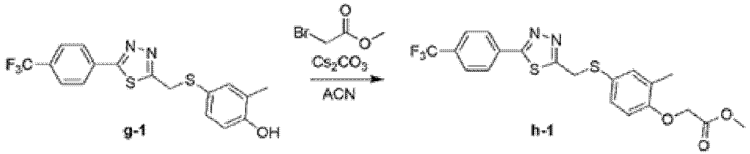

Compound g-1 (0.3 g, 0.78 mmol) was dissolved in acetonitrile (5 mL), and cesium carbonate (0.38 g, 1.17 mmol) and methyl 2-bromoacetate (0.9 mL, 0.94 mmol) were added dropwise, followed by stirring at room temperature for 1 hour and 30 minutes. After the reaction was completed, it was concentrated under reduced pressure to remove acetonitrile, washed with water and saline solution, extracted with EtOAc, and dried over anhydrous Na₂SO₄. The obtained residue was concentrated under reduced pressure, purified by MPLC (silica gel, 5-20%, EtOAc/Hexane), and concentrated under reduced pressure to obtain Compound h-1 (0.32 g, 96% yield).

### Step 8: Preparation of 2-(2-methyl-4-(((5-(4-(trifluoromethyl)phenyl)-1,3,4-thiadiazol-2-yl)methyl)thio)phenoxy)acetic acid (1)

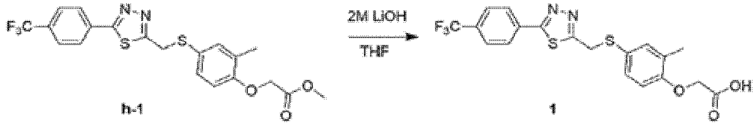

Compound h-1 (0.32 g, 0.7 mmol) was dissolved in tetrahydrofuran, then 2 M lithium hydroxide aqueous solution (1.5 mL) was added at 0°C, followed by stirring at room temperature for 1 hour. After the reaction was completed, it was quenched with water and acidified with 1N HCl. Afterwards, it was concentrated under reduced pressure to remove the solvent, extracted with EtOAc, and dried over anhydrous Na₂SO₄ to obtain Compound 1 (0.25 g, yield 93%).

ESI LC/MS: m/z calcd. for C₁₉H₁₆F₃N₂O₃S₂ [M+H]⁺: 441.06; found 441.1. ¹H NMR (DMSO-d₆, 400MHz) δ8.14 (d, *J =* 8.08 Hz, 2H), 7.88 (d, *J =* 8.2 Hz, 2H), 7.28 (s, 1H), 7.21 (dd, *J =* 8.44, 1.92 Hz, 1H), 6.77 (d, *J =* 8.56 Hz, 1H), 4.67 (s, 2H), 4.64 (s, 2H), 2.13 (s, 3H). ¹³C NMR (DMSO-d₆, 400MHz) δ170.69, 170.29, 167.41, 156.01, 133.83, 133.44, 130.34, 128.61, 127.44, 126.59, 126.55, 123.78, 112.28, 64.88, 33.03, 16.12.

### <Example 2> Preparation of 2-(2-methyl-4-(((5-(4-(trifluoromethyl)phenyl)-1,3,4-thiadiazol-2-yl)methyl)thio)phenoxy)propanoic acid

### Step 1: Preapration of ethyl 2-(2-methyl-4-(((5-(4-(trifluoromethyl)phenyl)-1,3,4-thiadiazol-2-yl)methyl)thiophenoxy)propanoate (i-1)

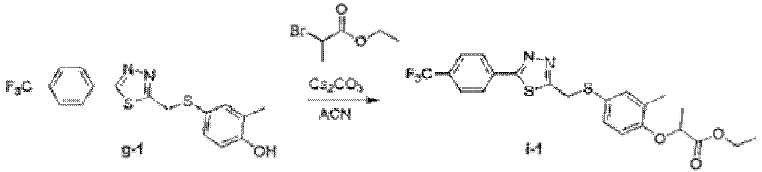

Compound i-1 (59 mg, 94% yield) was obtained in the same manner as in Step 7 of Example 1, except that ethyl 2-bromopropanoate was used instead of methyl 2-bromoacetate.

### Step 2: Preparation of 2-(2-methyl-4-(((5-(4-(trifluoromethyl)phenyl)-1,3,4-thiadiazol-2-yl)methyl)thiophenoxy)propanoic acid (2)

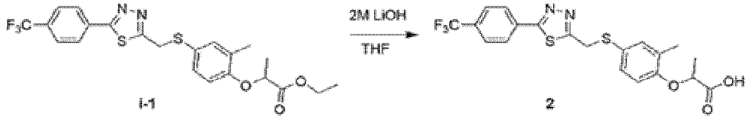

Compound 2 (46 mg, 83% yield) was obtained in the same manner as in Step 8 of Example 1, except that Compound i-1 was used instead of Compound h-1.

ESI LC/MS: m/z calcd. for C₂₀H₁₈F₃N₂O₃S₂ [M+H]⁺: 455.07; found 455.1. ¹H NMR (CD₃OD, 400MHz) δ8.08 (d, *J =* 8.16 Hz, 2H), 7.80 (d, *J =* 8.24 Hz, 2H), 7.25 (s, 1H), 7.19 (dd, *J =* 8.44, 2.02 Hz, 1H), 6.89 (d, *J =* 8.48 Hz, 1H), 4.78 (q, *J =* 6.76 Hz, 1H), 4.46 (s, 2H), 2.19 (s, 3H), 1.59 (d, *J =* 6.8 Hz, 3H), ¹³C NMR (CD₃OD, 100MHz) δ175.57, 172.53, 169.61, 157.85, 136.35, 132.69, 132.56, 129.89, 129.50, 127.46, 127.42, 125.23, 113.57, 73.65, 35.09, 19.01, 16.47.

### <Example 3> Preparation of 2-methyl-2-(2-methyl-4-(((5-(4-(trifluoromethyl)phenyl)-1,3,4-thiadiazol-2-yl)methyl)thio)phenoxy)propanoic acid

### Step 1: Preparation of ethyl 2-methyl-2-(2-methyl-4-(((5-(4-(trifluoromethyl)phenyl)-1,3,4-thiadiazol-2-yl)methyl)thio)phenoxy)propanoate (j-1)

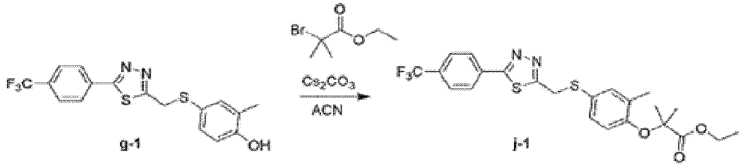

Compound j-1 (18 mg, 28% yield) was obtained in the same manner as in Step 7 of Example 1, except that ethyl-2-bromo-2-methylpropanoate was used instead of methyl 2-bromoacetate.

### Step 2: Preparation of 2-methyl-2-(2-methyl-4-(((5-(4-(trifluoromethyl)phenyl)-1,3,4-thiadiazol-2-yl)methyl)thio)phenoxy)propanoic acid (3)

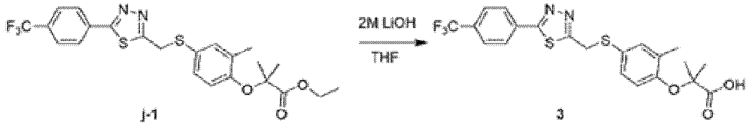

Compound 3 (9 mg, 64% yield) was obtained in the same manner as in Step 8 of Example 1, except that Compound j-1 was used instead of Compound h-1.

ESI LC/MS: m/z calcd. for C₂₁H₂₀F₃N₂O₃S₂ [M+H]⁺: 469.09; found 469.12. ¹H NMR (CD₃OD, 400MHz) δ8.11 (d, *J =* 8.12 Hz, 2H), 7.82 (d, *J =* 8.24 Hz, 2H), 7.26 (s, 1H), 7.16 (dd, *J =* 8.48, 1.99 Hz, 1H), 6.69 (d, *J =* 8.52 Hz, 1H), 4.51 (s, 2H), 2.16 (s, 3H), 1.56 (s, 6H), ¹³C NMR (CD₃OD, 100MHz) δ177.71, 172.53, 169.65, 155.73, 136.30, 131.93, 131.88, 129.51, 127.49, 127.45, 125.90, 118.15, 80.33, 34.91, 25.83, 16.87

### <Example 4> Preparation of 2-(2-methyl-4-(((5-(4-(trifluoromethyl)phenyl)-1,3,4-thiadiazol-2-yl)methyl)thio)phenoxy)acetonitrile

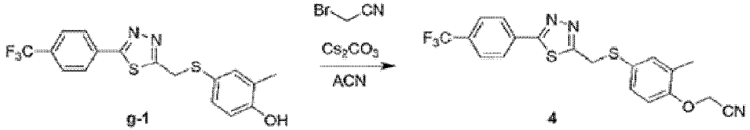

Compound 4 (0.163 g, 67% yield) was obtained in the same manner as in Step 7 of Example 1, except that 2-bromoacetonitrile was used instead of methyl 2-bromoacetate.

¹H NMR (CDCl₃, 400 MHz) δ8.04 (d, *J =* 8.1 Hz, 2H), 7.72 (d, *J =* 8.2 Hz, 2H), 7.27-7.24 (m, 2H), 6.82 (d, *J=* 8.0 Hz, 1H), 4.76 (s, 2H), 4.45 (s, 2H), 2.20 (s, 3H).

### <Example 5> Preparation of 2-(((4-((2H-tetrazol-5-yl)methoxy)-3-methylphenyl)thio)methyl)-5-(4-(trifluoromethyl)phenyl)-1,3,4-thiadiazole

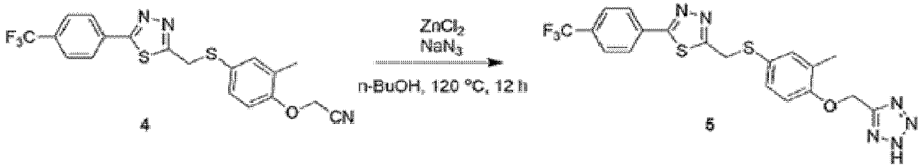

Compound 4 (21 mg, 0.050 mmol), NaN₃ (3.9 mg, 0.060 mmol), ZnCl₂ (6.8 mg, 0.050 mmol) were dissolved in 0.4 mL of n-butanol and reacted at 120°C for 12 hours. The reaction mixture was distilled under reduced pressure and separated using Prep-HPLC to obtain Compound 5 (9.5 mg, 41% yield).

¹H NMR (CDCl₃, 400 MHz) δ7.99 (d, *J =* 8.1 Hz, 2H), 7.72 (d, *J =* 8.2 Hz, 2H), 7.13-7.12 (m, 2H), 6.82 (d, *J =* 8.0 Hz, 1H), 5.38 (s, 2H), 4.37 (s, 2H), 1.99 (s, 3H).

### <Example 6> Preparation of N-hydroxy-2-(2-methyl-4-(((5-(4-(trifluoromethyl)phenyl)-1,3,4-thiadiazol-2-yl)methyl)thio)phenoxy)acetamide

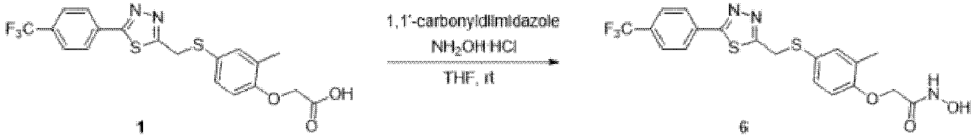

Compound 1 (3.7 mg, 0.008 mmol) was dissolved in 1 mL of THF, then 1,1'-carbonyldiimidazole (3.4 mg, 0.021 mmol) was added, followed by a reaction at room temperature for 1 hour. Hydroxylamine hydrochloride (1.5 mg, 0.021 mmol) was added to the reaction mixture, followed by a reaction at room temperature for another 16 hours. Potassium hydrogen sulfate (5.7 mg, 0.042 mmol) was added to the mixture, then 0.5 mL of distilled water was added, followed by a reaction at room temperature for another 1 hour. The solvent was removed through reduced pressure distillation and separated using Prep-HPLC to obtain Compound 6 (2.7 mg, 69% yield).

¹H NMR (CD₃OD, 400 MHz) δ8.14 (d, *J =* 7.8 Hz, 2H), 7.85 (d, *J =* 8.1 Hz, 2H), 7.30 (s, 1H), 7.26 (d, *J =* 8.0 Hz, 1H), 6.83 (d, *J* = 7.9 Hz, 1H), 4.56 (s, 2H), 4.53 (s, 2H), 2.24 (s, 3H).

### <Example 7> Preparation of N-methoxy-2-(2-methyl-4-(((5-(4-(trifluoromethyl)phenyl)-1,3,4-thiadiazol-2-yl)methyl)thio)phenoxy)acetamide

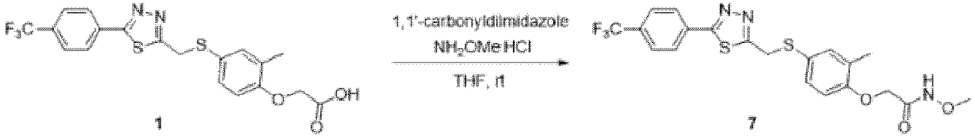

Compound 7 (9.2 mg, yield 85%) was obtained in the same manner as in Example 6, except that O-methylhydroxylamine hydrochloride was used instead of hydroxylamine hydrochloride.

¹H NMR (CD₃OD, 400 MHz) δ8.12 (d, *J =* 7.8 Hz, 2H), 7.84 (d, *J =* 8.0 Hz, 2H), 7.30 (s, 1H), 7.26 (d, *J =* 8.3 Hz, 1H), 6.82 (d, *J =* 8.2 Hz, 1H), 4.56 (s, 2H), 4.52 (s, 2H), 3.71 (s, 3H), 2.24 (s, 3H).

### <Example 8> Preparation of N-cyano-2-(2-methyl-4-(((5-(4-(trifluoromethyl)phenyl)-1,3,4-thiadiazol-2-yl)methyl)thio)phenoxy)acetamide

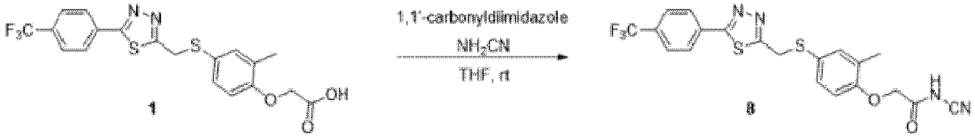

Compound 8 (14 mg, 42% yield) was obtained in the same manner as in Example 6, except that cyanamide was used instead of hydroxylamine hydrochloride.

¹H NMR (CDCl₃, 400 MHz) δ8.11 (d, *J =* 8.0 Hz, 2H), 7.83 (d, *J =* 8.3 Hz, 2H), 7.30-7.22 (m, 2H), 6.81-6.75 (m, 1H), 4.69 (s, 2H), 4.51 (s, 2H), 2.23 (s, 3H).

### <Example 9> Preparation of 2-(4-(((5-(4-fluorophenyl)-1,3,4-thiadiazol-2-yl)methyl)thio)-2-methylphenoxy)acetic acid

### Step 1: Preparation of 4-fluorobenzohydrazide (b-2)

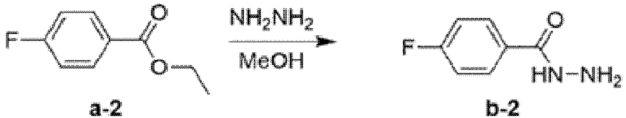

### Compound b-2 (1.03 g, 67% yield) was obtained in the same manner as in Step 1 of Example 1, except that Compound a-2 was used instead of Compound a-1.

### Step 2: Preparation of ethyl 2-(2-(4-fluorobenzoyl)hydrazinyl)-2-oxoacetate (c-2)

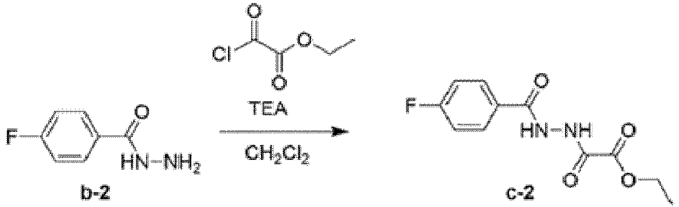

Compound c-2 (0.236 g, 93% yield) was obtained in the same manner as in step 2 of Example 1, except that Compound b-2 was used instead of Compound b-1.

### Step 3: Preparation of ethyl 5-(4-fluorophenyl)-1,3,4-thiadiazole-2-carboxylate (d-2)

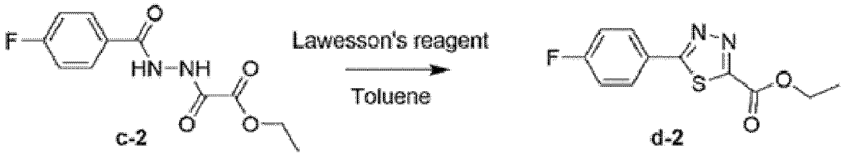

Compound d-2 (0.256 g, 78% yield) was obtained in the same manner as in Step 3 of Example 1, except that Compound c-2 was used instead of Compound c-1.

### Step 4: Preparation of (5-(4-(trifluoromethoxy)phenyl)-1,3,4-thiadiazol-2-yl)methanol (e-2)

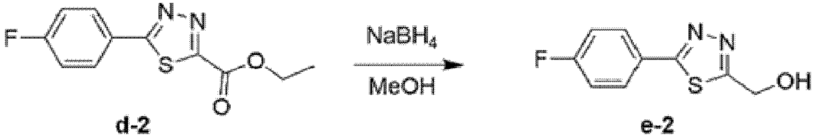

Compound e-2 (0.185 g, 88% yield) was obtained in the same manner as in Step 4 of Example 1, except that Compound d-2 was used instead of Compound d-1.

### Step 5: Preparation of (5-(4-fluorophenyl)-1,3,4-thiadiazol-2-yl)methanol (f-2)

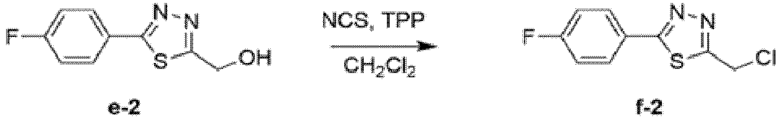

Compound f-2 (0.178 g, 78% yield) was obtained in the same manner as in Step 5 of Example 1, except that Compound e-2 was used instead of Compound e-1.

### Step 6: Preparation of 4-(((5-(4-fluorophenyl)-1,3,4-thiadiazol-2-yl)methyl)thio-2-methylphenol (g-2)

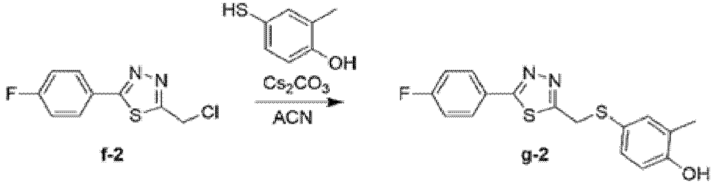

Compound g-2 (0.332 g, 97% yield) was obtained in the same manner as in Step 6 of Example 1, except that Compound f-2 was used instead of Compound f-1.

### Step 7: Preparation of methyl 2-(4-(((5-(4-fluorophenyl)-1,3,4-thiadiazol-2-yl)methyl)thio)-2-methylphenoxy)acetate (h-2)

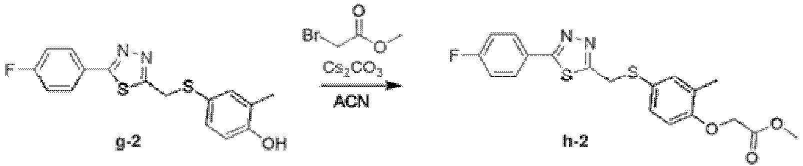

Compound h-2 (0.340 g, 84% yield) was obtained in the same manner as in Step 7 of Example 1, except that Compound g-2 was used instead of Compound g-1.

### Step 8: Preparation of 2-(4-(((5-(4-fluorophenyl)-1,3,4-thiadiazol-2-yl)methyl)thio)-2-methylphenoxy)acetic acid (9)

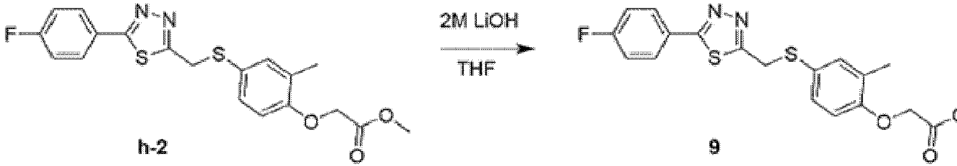

Compound 9 (0.363 g, 93% yield) was obtained in the same manner as in Step 8 of Example 1, except that Compound h-2 was used instead of Compound h-1.

ESI LC/MS: m/z calcd. for C₁₈H₁₆FN₂O₃S₂ [M+H]⁺: 391.06; found 391. ¹H NMR (400 MHz, CDCl₃) δ7.84 (dd, *J =* 8.5, 5.3 Hz, 2H), 7.13 - 7.07 (m, 4H), 6.54 (d, *J =* 8.4 Hz, 1H), 4.47 (s, 2H), 4.33 (s, 2H), 2.12 (s, 3H); ¹³C NMR (100 MHz, CDCl₃) δ171.38, 169.49, 168.57, 164.44 (d, JCF = 251.2 Hz), 156.14, 134.76, 130.75, 129.85, 128.60, 126.11, 124.51, 116.28, 112.21, 60.50, 34.28, 16.13, 14.20.

### <Example 10> Preparation of 2-(4-(((5-(4-fluorophenyl)-1,3,4-thiadiazol-2-yl)methyl)thio)-2-methylphenoxy)propanoic acid

### Step 1: Preparation of ethyl 2-(4-(((5-(4-fluorophenyl)-1,3,4-thiadiazol-2-yl)methyl)thio)-2-methylphenoxy)propanoate(i-2)

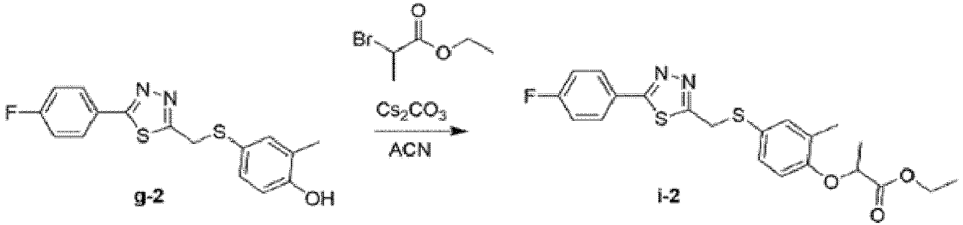

Compound i-2 (0.320 g, 74% yield) was obtained in the same manner as in Step 7 of Example 1, except that Compound g-2 was used instead of Compound g-1 and ethyl 2-bromopropanoate was used instead of methyl 2-bromoacetate.

### Step 2: Preparation of 2-(4-(((5-(4-fluorophenyl)-1,3,4-thiadiazol-2-yl)methyl)thio)-2-methylphenoxy)propanoic acid (10)

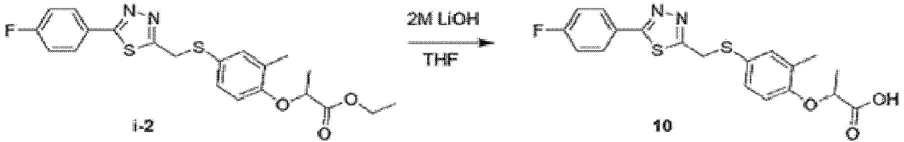

Compound 10 (0.259 g, 64% yield) was obtained in the same manner as in Step 8 of Example 1, except that Compound i-2 was used instead of Compound h-1.

ESI LC/MS: m/z calcd. for C₁₉H₁₈FN₂O₃S₂ [M+H]⁺: 405.08; found 405. ¹H NMR (DMSO-d₆, 400 MHz) δ 8.01 - 7.97 (m, 2H), 7.38 (t, *J =* 8.8 Hz, 2H), 7.28 (s, 1H), 7.20 (dd, *J* = 8.5, 1.8 Hz, 1H), 6.72 (d, *J =* 8.6 Hz, 1H), 4.77 (q, *J =* 6.7 Hz, 1H), 4.61 (s, 2H), 2.13 (s, 3H), 1.49 (d, *J =* 6.7 Hz, 3H). ¹³C NMR (DMSO-d₆, 100 MHz) δ 173.56, 169.86, 167.97, 164.19 (d, JCF = 248.0 Hz), 156.09, 134.09, 130.45, 127.87, 126.64, 123.96, 116.91, 112.98, 72.42, 49.07, 33.30, 18.83, 16.41.

### <Example 11> Preparation of 2-(4-(((5-(4-fluorophenyl)-1,3,4-thiadiazol-2-yl)methyl)thio)-2-methylphenoxy)-2-methylpropanoic acid

### Step 1: Preparation of ethyl 2-(4-(((5-(4-fluorophenyl)-1,3,4-thiadiazol-2-yl)methyl)thio)-2-methylphenoxy)-2-methyl propanoate (j-2)

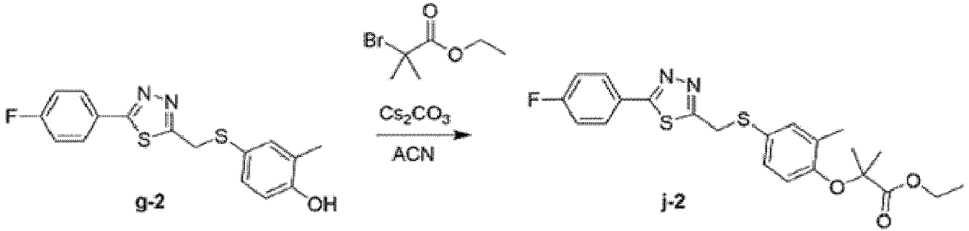

Compound j-2 (0.174 g, 39% yield) was obtained in the same manner as in Step 7 of Example 1, except that Compound g-2 was used instead of Compound g-1, and ethyl 2-bromo-2-methylpropanoate was used instead of methyl 2-bromoacetate.

### Step 2: Preparation of 2-(4-(((5-(4-fluorophenyl)-1,3,4-thiadiazol-2-yl)methyl)thio)-2-methylphenoxy)-2-methylpropanoic acid (11)

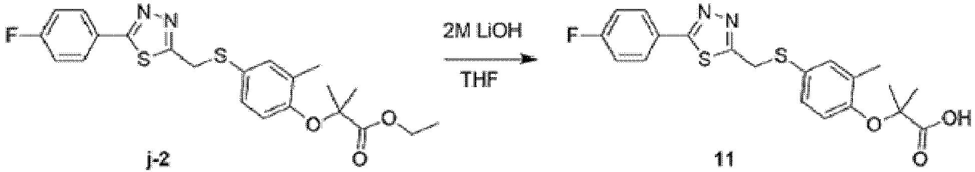

Compound 11 (0.167 g, 40% yield) was obtained in the same manner as in Step 8 of Example 1, except that Compound j-2 was used instead of Compound h-1.

ESI LC/MS: m/z calcd. for C₂₀H₂₀FN₂O₃S₂ [M+H]⁺: 419.09; found 419. ¹H NMR (400 MHz, CDCl₃) δ7.89 (dd, *J* = 8.4, 5.2 Hz, 2H), 7.21 (s, 1H), 7.15 (t, *J =* 8.5 Hz, 2H), 7.10 (d, *J =* 8.0 Hz, 1H), 6.70 (d, *J =* 8.5 Hz, 1H), 4.40 (s, 2H), 2.17 (s, 3H), 1.25 (s, 3H). ¹³C NMR (CDCl₃, 100 MHz) δ171.34, 169.42, 168.50, 164.45 (d, JCF = 250.9 Hz), 153.60, 134.55, 131.01, 129.87, 126.25, 125.33, 117.79, 116.28, 79.52, 60.49, 34.15, 25.27, 16.75.

### <Example 12> Preparation of 2-(4-(((5-(3,4-difluorophenyl)-1,3,4-thiadiazol-2-yl)methyl)thio)-2-methylphenoxy)acetic acid

### Step 1: Preparation of 3,4-difluorobenzohydrazide (b-3)

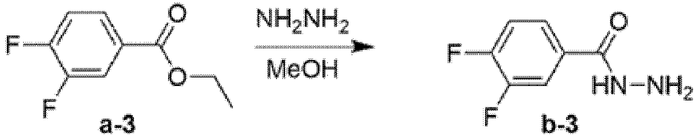

Compound b-3 (0.34 g, 73.5% yield) was obtained in the same manner as in Step 1 of Example 1, except that Compound a-3 was used instead of Compound a-1.

### Step 2: Preparation of ethyl 2-(2-(3,4-difluorobenzoyl)hydrazinyl)-2-oxoacetate (c-3)

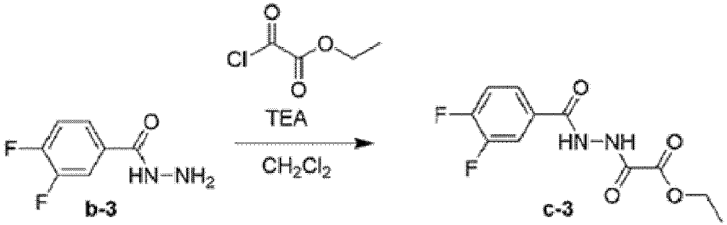

Compound c-3 (0.36 g, 67% yield) was obtained in the same manner as in Step 2 of Example 1, except that Compound b-3 was used instead of Compound b-1.

### Step 3: Preparation of ethyl 5-(3,4-difluorophenyl)-1,3,4-thiadiazole-2-carboxylate (d-3)

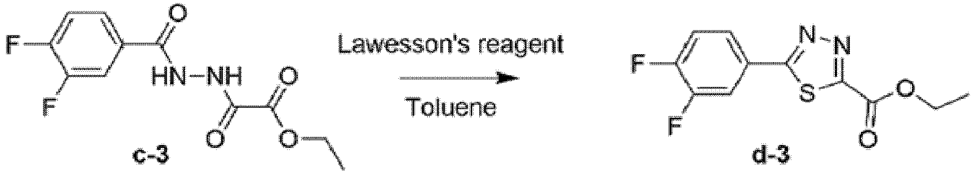

Compound d-3 (54 mg, 34% yield) was obtained in the same manner as in Step 3 of Example 1, except that Compound c-3 was used instead of Compound c-1.

### Step 4: Preparation of (5-(3,4-difluorophenyl)-1,3,4-thiadiazol-2-yl)methanol (e-3)

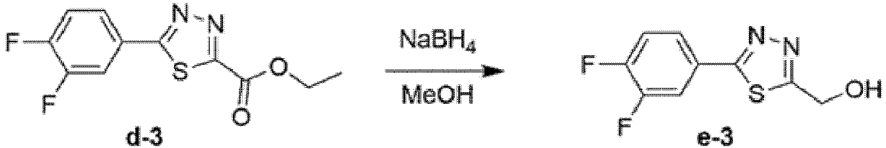

Compound e-3 (45 mg, 99% yield) was obtained in the same manner as in Step 4 of Example 1, except that Compound d-3 was used instead of Compound d-1.

### Step 5: Preparation of 2-(chloromethyl)-5-(3,4-difluorophenyl)-1,3,4-thiadiazole (f-3)

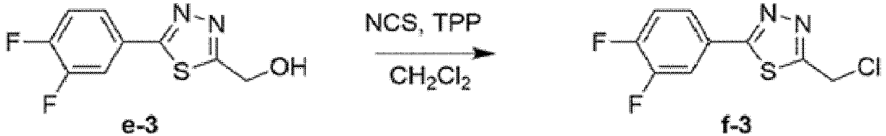

Compound f-3 (36 mg, 74% yield) was obtained in the same manner as in Step 5 of Example 1, except that Compound e-3 was used instead of Compound e-1.

### Step 6: Preparation of 4-(((5-(3,4-difluorophenyl)-1,3,4-thiadiazol-2-yl)methyl)thio)-2-methylphenol (g-3)

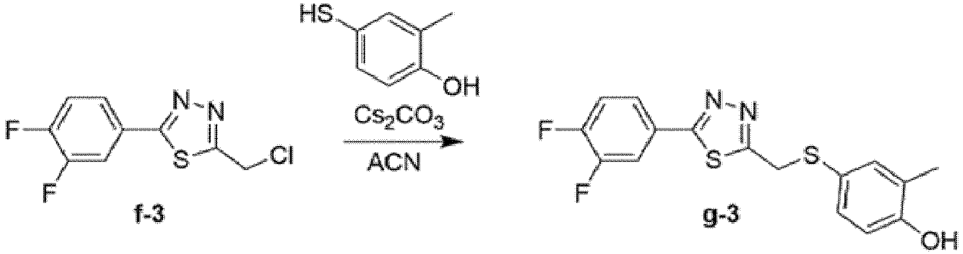

Compound g-3 (50 mg, 98% yield) was obtained in the same manner as in Step 6 of Example 1, except that Compound f-3 was used instead of Compound f-1.

### Step 7: Preparation of methyl 2-(4-(((5-(3,4-difluorophenyl)-1,3,4-thiadiazol-2-yl)methyl)thio)-2-methyl)phenoxy)acetate (h-3)

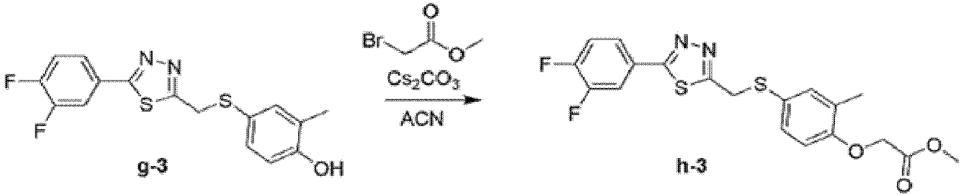

Compound h-3 (64 mg, 99% yield) was obtained in the same manner as in Step 7 of Example 1, except that Compound g-3 was used instead of Compound g-1.

### Step 8: Preparation of 2-(4-(((5-(3,4-difluorophenyl)-1,3,4-thiadiazol-2-yl)methyl)thio)-2-methylphenoxy)acetic acid (12)

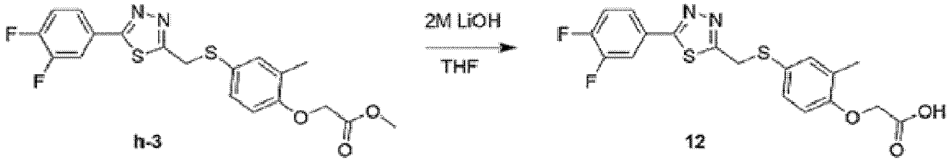

Compound 12 (25 mg, 39.8% yield) was obtained in the same manner as in Step 8 of Example 1, except that Compound h-3 was used instead of Compound h-1.

ESI LC/MS: m/z calcd. for C₁₈H₁₅F₂N₂O₃S₂ [M+H]⁺: 409.05; found 409. ¹H NMR (DMSO, 400MHz) δ 8.07 (s, 1H), 7.81 (s, 1H), 7.64 (d, *J =* 7.28 Hz, 1H), 7.25 (s, 1H), 7.19 (s, 1H), 6.71 (s, 1H), 4.62 (s, 2H), 4.43 (s, 2H), 2.13 (d, *J =* 7.3 Hz, 3H).

### <Example 13> Preapration of 2-(4-(((5-(3,4-difluorophenyl)-1,3,4-thiadiazol-2-yl)methyl)thio)-2-methylphenoxy)propanoic acid

### Step 1: Preparation of ethyl 2-(4-(((5-(3,4-difluorophenyl)-1,3,4-thiadiazol-2-yl)methyl)thio)-2-methylphenoxy)propanoate (i-3)

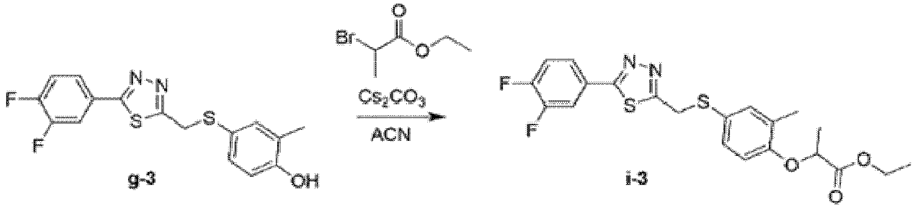

Compound i-3 (67 mg, 97% yield) was obtained in the same manner as in Step 7 of Example 1, except that Compound g-3 was used instead of Compound g-1, and 2-bromopropanoate was used instead of methyl 2-bromoacetate.

### Step 2: Preparation of 2-(4-(((5-(3,4-difluorophenyl)-1,3,4-thiadiazol-2-yl)methyl)thio)-2-methylphenoxy)propanoic acid (13)

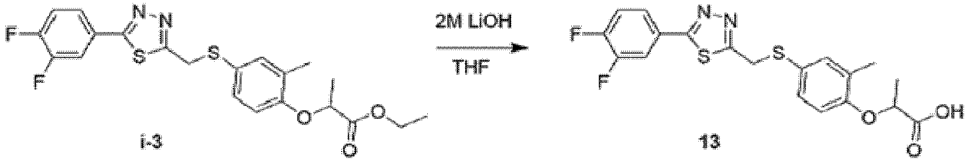

Compound 13 (3 mg, 5% yield) was obtained in the same manner as in Step 8 of Example 1, except that Compound i-3 was used instead of Compound h-1.

ESI LC/MS: m/z calcd. for C₁₉H₁₈FN₂O₃S₂ [M+H]⁺: 423.07; found 423. ¹H NMR (CD₃OD, 400MHz) δ 7.93 (m, 1H), 7.74 (s, 1H), 7.47 (m, 1H), 7.27 (s, 1H), 7.22 (d, *J* = 8.44 Hz, 1H), 6.73 (d, *J =* 8.40 Hz, 1H), 4.47 (s, 2H), 2.21 (s, 3H), 1.61 (d, *J =* 6.72 Hz, 3H).

### <Example 14> Preparation of 2-(4-(((5-(3,4-difluorophenyl)-1,3,4-thiadiazol-2-yl)methyl)thio)-2-methylphenoxy)-2-methylpropanoic acid

### Step 1: Preparation of ethyl 2-(4-(((5-(3,4-difluorophenyl)-1,3,4-thiadiazol-2-yl)methyl)thio)-2-methylphenoxy)-2-methylpropanoate (j-3)

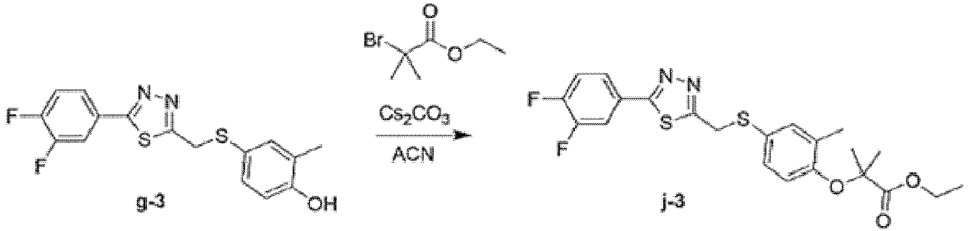

Compound j-3 (33 mg, 50% yield) was obtained in the same manner as in Step 7 of Example 1, except that Compound g-3 was used instead of Compound g-1, and ethyl 2-bromo-2-methylpropanoate was used instead of methyl 2-bromoacetate.

### Step 2: Preparation of 2-(4-(((5-(3,4-difluorophenyl)-1,3,4-thiadiazol-2-yl)methyl)thio)-2-methylphenoxy)-2-methylpropanoic acid (14)

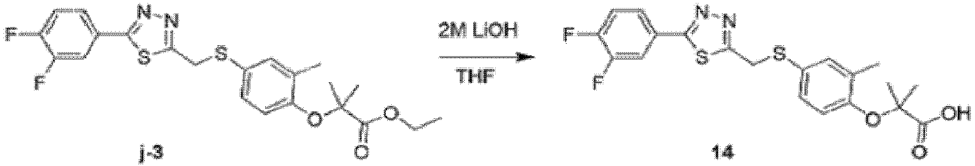

Compound 14 (12 mg, 38% yield) was obtained in the same manner as in Step 8 of Example 1, except that Compound j-3 was used instead of Compound h-1.

ESI LC/MS: m/z calcd. for C₁₉H₁₈FN₂O₃S₂ [M+H]⁺: 437.08; found 437.1. ¹H NMR (CD₃OD, 400MHz) δ 7.93 (m, 1H), 7.74 (m, 1H), 7.47 (m, 1H), 7.27 (s, 1H), 7.18 (dd, *J= 2.12* Hz, 6.40 Hz, 1H), 6.72 (d, *J =* 8.52 Hz, 1H), 4.49 (s, 2H), 2.17 (s, 3H), 1.58 (s, 6H). ¹³C NMR (CD₃OD, 100 MHz) δ15.37, 24.33, 33.37, 78.81, 116.08, 116.28, 116.61, 118.07, 118.25, 124.39, 124.71, 130.38, 134.76, 154.20, 170.68, 176.20.

### <Example 15> Preparation of 2-(2-methyl-4-(((5-(3,4,5-trifluorophenyl)-1,3,4-thiadiazol-2-yl)methyl)thio)phenoxy)acetic acid

### Step 1: Preparation of 3,4,5-trifluorobenzohydrazide (b-4)

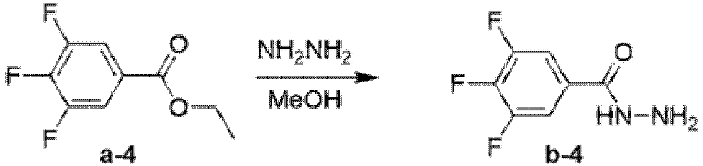

Compound b-4 (0.74 g, 39% yield) was obtained in the same manner as in Step 1 of Example 1, except that Compound a-4 was used instead of Compound a-1.

### Step 2: Preparation of ethyl 2-oxo-2-(2-(3,4,5-trifluorobenzoyl)hydrazinyl)acetate (c-4)

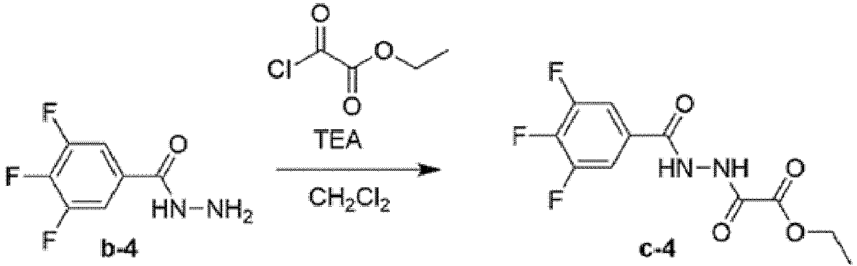

Compound c-4 (0.209 g, 72% yield) was obtained in the same manner as in Step 2 of Example 1, except that Compound b-4 was used instead of Compound b-1.

### Step 3: Preparation of ethyl 5-(3,4,5-trifluorophenyl)-1,3,4-thiadiazole-2-carboxylate (d-4)

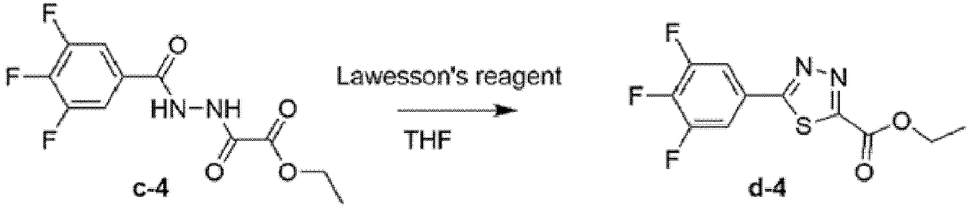

Compound d-4 (0.21 g, 56% yield) was obtained in the same manner as in Step 3 of Example 1, except that Compound c-4 was used instead of Compound c-1.

### Step 4: Preparation of (5-(3,4,5-trifluorophenyl)-1,3,4-thiadiazol-2-yl)methanol (e-4)

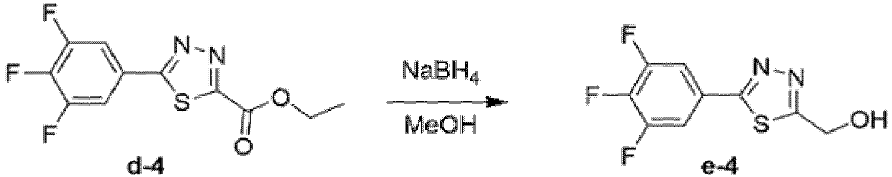

Compound e-4 (0.24 g, 99% yield) was obtained in the same manner as in Step 4 of Example 1, except that Compound d-4 was used instead of Compound d-1.

### Step 5: Preparation of 2-(chloromethyl)-5-(3,4,5-trifluorophenyl)-1,3,4-thiadiazole (f-4)

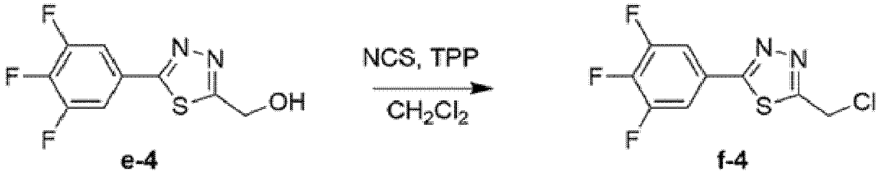

Compound f-4 (0.18 g, 68% yield) was obtained in the same manner as in Step 5 of Example 1, except that Compound e-4 was used instead of Compound e-1.

### Step 6: Preparation of 2-methyl-4-(((5-(3,4,5-trifluorophenyl)-1,3,4-thiadiazol-2-yl)methyl)thio)phenol (g-4)

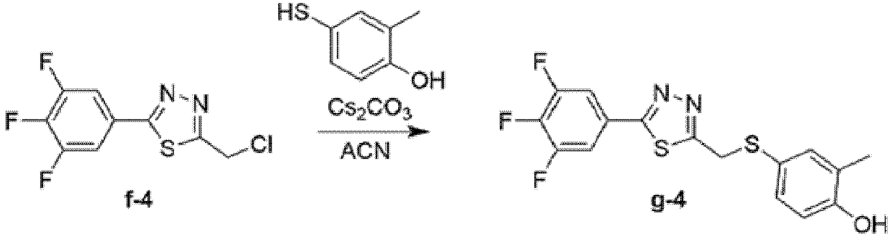

Compound g-4 (0.368 g, 99% yield) was obtained in the same manner as in Step 6 of Example 1, except that Compound f-4 was used instead of Compound f-1.

### Step 7: Preparation of methyl 2-(2-methyl-4-(((5-(3,4,5-trifluorophenyl)-1,3,4-thiadiazol-2-yl)methyl)thio)phenoxy)acetate (h-4)

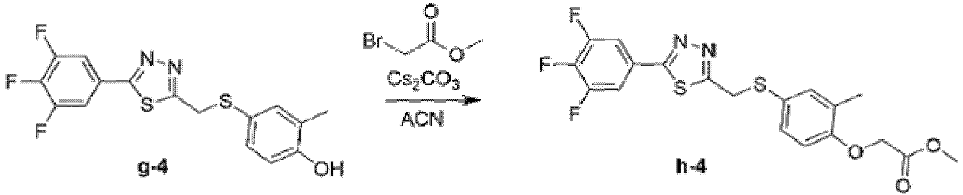

Compound h-4 (0.247 g, 56% yield) was obtained in the same manner as in Step 7 of Example 1, except that Compound g-4 was used instead of Compound g-1.

### Step 8: Preparation of 2-(2-methyl-4-(((5-(3,4,5-trifluorophenyl)-1,3,4-thiadiazol-2-yl)methyl)thio)phenoxy)acetic acid

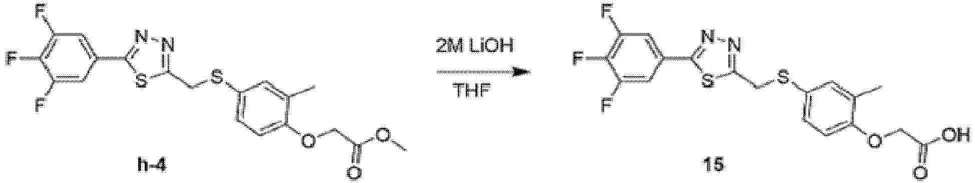

Compound 15 (64 mg, 15% yield) was obtained in the same manner as in Step 8 of Example 1, except that Compound h-4 was used instead of Compound h-1.

ESI LC/MS: m/z calcd. for C₁₈H₁₄F₃N₂O₃S₂ [M+H]⁺: 427.04; found 427. ¹H NMR (CD₃OD, 400 MHz) δ 7.67 (t, *J* = 7.4 Hz, 2H), 7.15 - 7.09 (m, 2H), 6.63 (d, *J* = 8.3 Hz, 1H), 4.37 (s, 2H), 3.55 (s, 2H), 2.10 (s, 3H).

### <Example 16> Preparation of 2-(4-(((5-(3-chloro-4-fluorophenyl)-1,3,4-thiadiazol-2-yl)methyl)thio)-2-methylphenoxy)acetic acid

### Step 1: Preparation of 3-chloro-4-fluorobenzohydrazide (b-5)

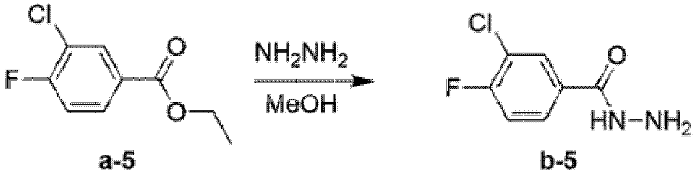

Compound b-5 (0.60 g, 88% yield) was obtained in the same manner as in Step 1 of Example 1, except that Compound a-5 was used instead of Compound a-1.

### Step 2: Preparation of ethyl 2-(2-(3-chloro-4-fluorobenzoyl)hydrazinyl)-2-oxoacetate (c-5)

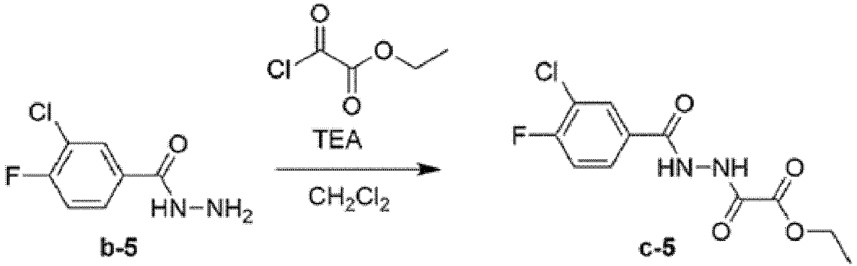

Compound c-5 (0.60 g, 88% yield) was obtained in the same manner as in Step 2 of Example 1, except that Compound b-5 was used instead of Compound b-1.

### Step 3: Preparation of ethyl 5-(3-chloro-4-fluorophenyl)-1,3,4-thiadiazole-2-carboxylate (d-5)

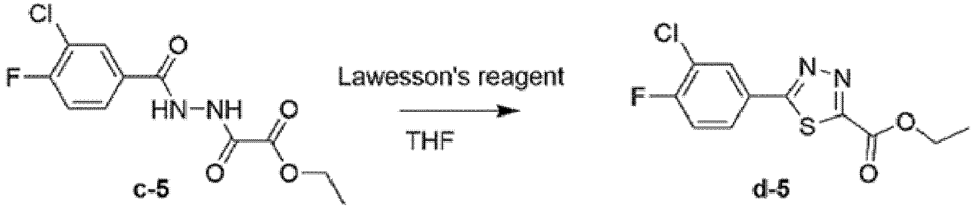

Compound d-5 (0.556 g, 60% yield) was obtained in the same manner as in Step 3 of Example 1, except that Compound c-5 was used instead of Compound c-1.

### Step 4: Preparation of (5-(3-chloro-4-fluorophenyl)-1,3,4-thiadiazol-2-yl)methanol (e-5)

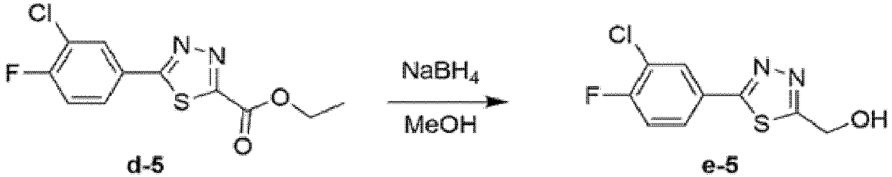

Compound e-5 (0.451 g, 87% yield) was obtained in the same manner as in Step 4 of Example 1, except that Compound d-5 was used instead of Compound d-1.

¹H NMR (400 MHz, CDCl₃) δ8.06 (dd, *J =* 6.9, 2.2 Hz, 1H), 7.84 (ddd, *J =* 8.6, 4.4, 2.2 Hz, 1H), 7.27 (t, *J =* 8.6 Hz, 1H), 5.14 (s, 2H), 2.72 (brs, 1H)

### Step 5: Preparation of 2-(3-chloro-4-fluorophenyl)-5-(chloromethyl)-1,3,4-thiadiazole (f-5)

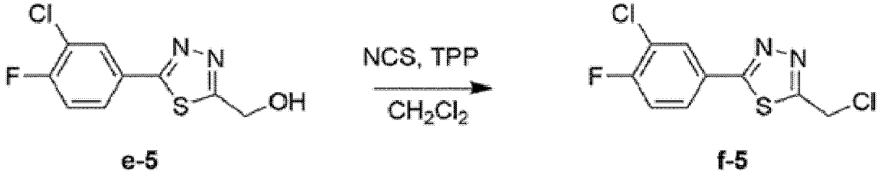

Compound f-5 (0.259 g, 53% yield) was obtained in the same manner as in Step 5 of Example 1, except that Compound e-5 was used instead of Compound e-1.

¹H NMR (400 MHz, CDCl₃) δ8.07 (dd, *J =* 6.8, 2.2 Hz, 1H), 7.84 (ddd, *J =* 8.6, 4.4, 2.3 Hz, 1H), 7.28 (t, *J =* 8.5 Hz, 1H), 4.99 (s, 2H)

### Step 6: Preapartion of 4-(((5-(3-chloro-4-fluorophenyl)-1,3,4-thiadiazol-2-yl)methyl)thio)-2-methylphenol (g- 5)

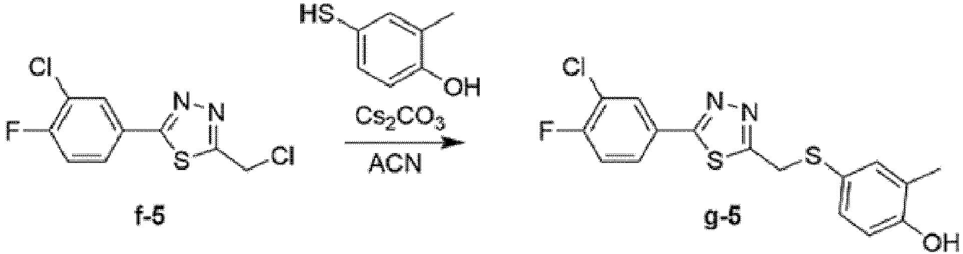

Compound g-5 (69 mg, 97% yield) was obtained in the same manner as in Step 6 of Example 1, except that Compound f-5 was used instead of Compound f-1.

### Step 7: Preparation of methyl 2-(4-(((5-(3-chloro-4-fluorophenyl)-1,3,4-thiadiazol-2-yl)methyl)thio)-2-methylphenoxy)acetate (h-5)

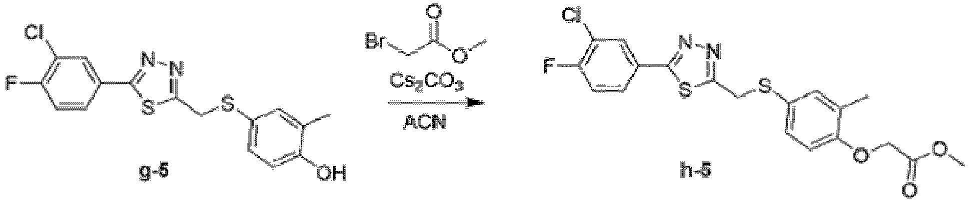

Compound h-5 (74 mg, 89% yield) was obtained in the same manner as in Step 7 of Example 1, except that Compound g-5 was used instead of Compound g-1.

¹H NMR (400 MHz, CDCl₃) δ8.01 (dd, *J* = 6.9, 2.2 Hz, 1H), 7.79 (ddd, *J =* 8.6, 4.5, 2.2 Hz, 1H), 7.26 - 7.21 (m, *J =* 5.3 Hz, 2H), 7.18 (dd, *J =* 8.4, 2.2 Hz, 1H), 6.61 (d, *J =* 8.5 Hz, 1H), 4.63 (s, 2H), 4.41 (s, 2H), 3.79 (s, 3H), 2.25 (s, 3H).

### Step 8: Preparation of 2-(4-(((5-(3-chloro-4-fluorophenyl)-1,3,4-thiadiazol-2-yl)methyl)thio)-2-methylphenoxy)acetic acid (16)

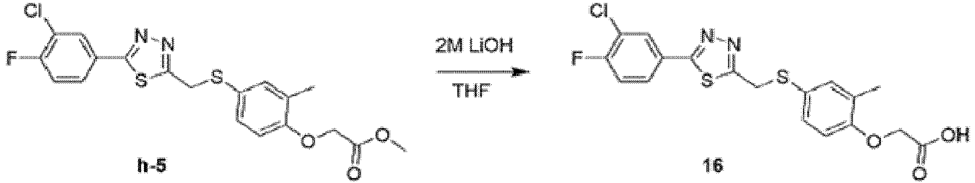

Compound 16 (44 mg, 88% yield) was obtained in the same manner as in Step 8 of Example 1, except that Compound h-5 was used instead of Compound h-1.

ESI LC/MS: m/z calcd. for C₁₈H₁₅ClFN₂O₃S₂ [M+H]⁺: 425.02; found 425. ¹H NMR (CD₃OD, 400MHz) δ8.12 - 8.04 (m, 1H), 7.91 - 7.82 (m, 1H), 7.41 (t, *J =* 8.8 Hz, 1H), 7.27 (s, 1H), 7.23 (d, *J =* 8.5 Hz, 1H), 6.76 (d, *J =* 8.4 Hz, 1H), 4.60 (s, 2H), 4.48 (s, 2H), 2.23 (s, 3H). ¹³C NMR (CD₃OD, 100 MHz) δ172.21, 170.76, 167.23, 159.62, 156.92, 134.75, 131.26, 129.36, 128.22, 128.16, 127.32, 123.52, 121.68, 117.35, 111.57, 65.45, 33.65, 14.94.

### <Example 17> Preparation of 2-(4-(((5-(3-chloro-4-fluorophenyl)-1,3,4-thiadiazol-2-yl)methyl)thio)-2-methylphenoxy)propanoic acid

### Step 1: Preparation of ethyl 2-(4-(((5-(3-chloro-4-fluorophenyl)-1,3,4-thiadiazol-2-yl)methyl)thio)-2-methylphenoxy)propanoate (i-5)

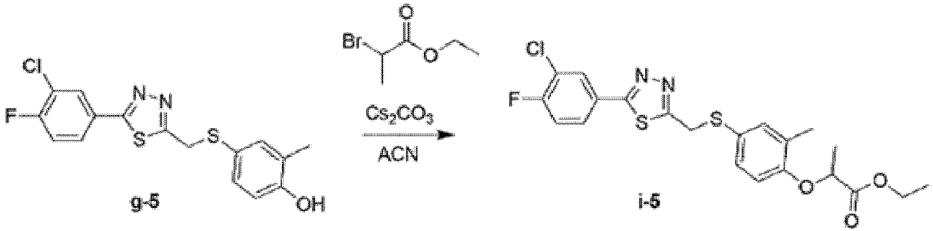

Compound i-5 (74.8 mg, 70.2% yield) was obtained in the same manner as in Step 7 of Example 1, except that Compound g-5 was used instead of Compound g-1, and the compound ethyl 2-bromopropanoate was used instead of methyl 2-bromoacetate.

¹H NMR (CDCl₃, 400 MHz) δ 8.01 (dd, *J* = 6.9, 2.2 Hz, 1H), 7.79 (ddd, *J =* 8.6, 4.5, 2.2 Hz, 1H), 7.25 - 7.21 (m, 2H), 7.15 (dd, *J =* 8.5, 2.2 Hz, 1H), 6.58 (d, *J =* 8.5 Hz, 1H), 4.69 (q, *J =* 6.8 Hz, 1H), 4.40 (s, 2H), 4.19 (qd, *J* = 7.1, 1.3 Hz, 2H), 2.23 (s, 3H), 1.62 (d, *J* = 6.8 Hz, 3H), 1.22 (t, *J =* 7.1 Hz, 3H).

### Step 2: Preparation of 2-(4-(((5-(3-chloro-4-fluorophenyl)-1,3,4-thiadiazol-2-yl)methyl)thio)-2-methylphenoxy)propanic acid (17)

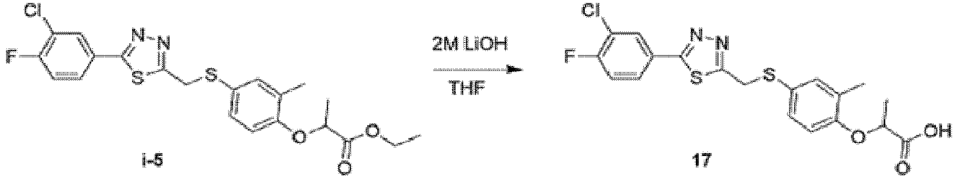

Compound 17 (33 mg, 85% yield) was obtained in the same manner as in Step 8 of Example 1, except that Compound i-5 was used instead of Compound h-1.

¹H NMR (CDCl₃, 400 MHz) δ7.99 (dd, *J=* 6.8, 1.9 Hz, 1H), 7.81 - 7.71 (m, 1H), 7.34 (brs, 1H), 7.23 (m, 2H), 7.15 (d, *J* = 8.4 Hz, 1H), 6.63 (d, *J =* 8.5 Hz, 1H), 4.73 (q, *J =* 6.7 Hz, 1H), 4.41 (s, 2H), 2.21 (s, 3H), 1.65 (d, *J* = 6.8 Hz, 3H). ¹³C NMR (CDCl₃, 100 MHz) δ176.05, 170.12, 167.17, 159.73, 155.89, 134.86, 130.73, 130.06, 128.93, 127.84, 127.22, 124.40, 122.28, 117.44, 112.53, 72.43, 34.34, 18.49, 16.25.

### <Example 18> Preparation of 2-(4-(((5-(3-chloro-4-fluorophenyl)-1,3,4-thiadiazol-2-yl)methyl)thio)-2-methylphenoxy)-2-methylpropanoic acid

### Step 1: Preparation of ethyl 2-(4-(((5-(3-chloro-4-fluorophenyl)-1,3,4-thiadiazol-2-yl)methyl)thio)-2-methylphenoxy)-2-methylpropanoate (j-5)

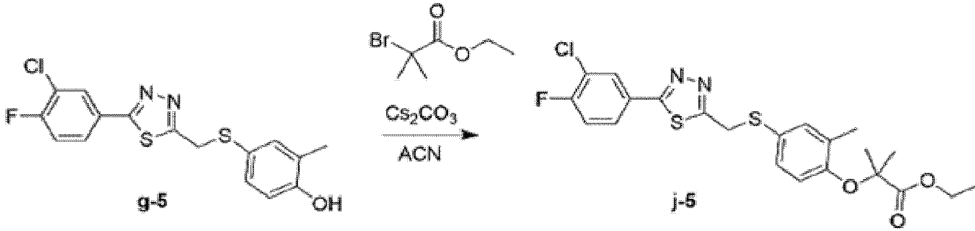

Compound j-5 (12 mg, 13% yield) was obtained in the same manner as in Step 7 of Example 1, except that Compound g-5 was used instead of Compound g-1, and the compound ethyl 2-bromo-2-methylpropanoate was used instead of methyl 2-bromoacetate.

### Step 2: Preparation of 2-(4-(((5-(3-chloro-4-fluorophenyl)-1,3,4-thiadiazol-2-yl)methyl)thio)-2-methylphenoxy)-2-methylpropanoic acid (18)

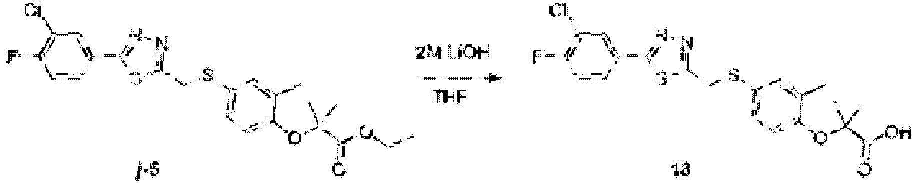

Compound 18 (3 mg, 26% yield) was obtained in the same manner as in Step 8 of Example 1, except that Compound j-5 was used instead of Compound h-1.

ESI LC/MS: m/z calcd. for C₂₀H₁₉ClFN₂O₃S₂ [M+H]⁺: 453.05; found 453. ¹H NMR (CD₃OD, 400 MHz) δ8.15 - 8.03 (m, 1H), 7.93 - 7.83 (m, 1H), 7.42 (t, *J =* 8.8 Hz, 1H), 7.28 (s, 1H), 7.18 (d, *J=* 8.3 Hz, 1H), 6.73 (d, *J =* 8.4 Hz, 1H), 4.49 (s, 2H), 2.18 (s, 3H), 1.58 (s, 6H). ¹³C NMR (CD₃OD, 400 MHz) δ170.69, 170.62, 167.27, 159.65 (d, *J =* 253.4 Hz), 154.39, 134.78, 130.45, 130.29, 129.40, 128.13, 127.32, 124.14, 121.69, 117.37, 116.67, 79.08, 33.44, 24.43, 15.37.

### <Example 19> Preparation of 2-(4-(((5-(3-fluoro-4-(trifluoromethyl)phenyl)-1,3,4-thiadiazol-2-yl)methyl)thio)-2-methylphenoxy)acetic acid

### Step 1: Preparation of 3-fluoro-4-(trifluoromethyl)benzohydrazide (b-6)

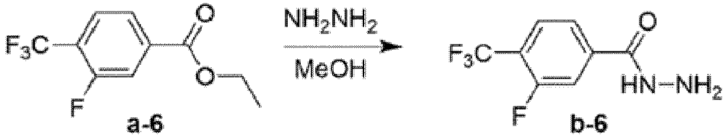

Compound b-6 (2.044 g, 92% yield) was obtained in the same manner as in Step 1 of Example 1, except that Compound a-6 was used instead of Compound a-1.

### Step 2: Preparation of ethyl 2-(2-(3-fluoro-4-(trifluoromethyl)benzoyl)hydrazinyl)-2-oxoacetate (c-6)

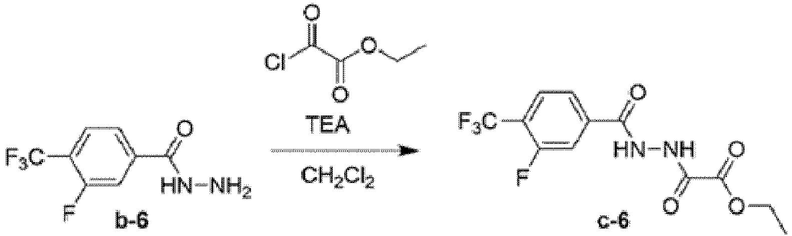

Compound c-6 (0.139 g, 43% yield) was obtained in the same manner as in Step 2 of Example 1, except that Compound b-6 was used instead of Compound b-1.

### Step 3: Preparation of ethyl 5-(3-fluoro-4-(trifluoromethyl)phenyl)-1,3,4-thiadiazole-2-carboxylate (d-6)

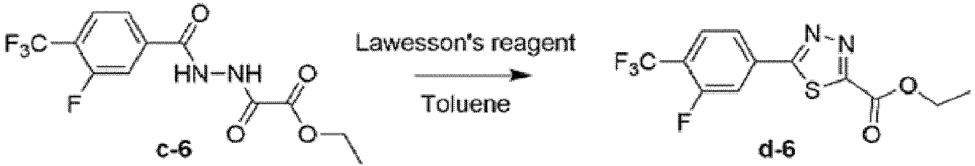

Compound d-6 (0.371 g, 89% yield) was obtained in the same manner as in Step 3 of Example 1, except that Compound c-6 was used instead of Compound c-1.

### Step 4: Preparation of (5-(3-fluoro-4-(trifluoromethyl)phenyl)-1,3,4-thiadiazol-2-yl)methanol (e-6)

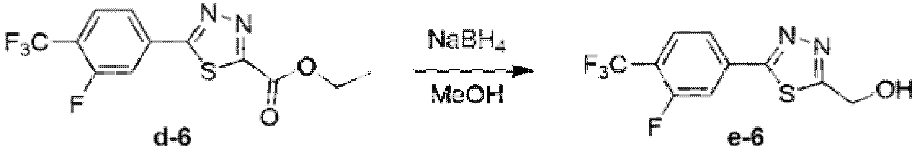

Compound e-6 (0.253 g, 91% yield) was obtained in the same manner as in Step 4 of Example 1, except that Compound d-6 was used instead of Compound d-1.

### Step 5: Preparation of 2-(chloromethyl)-5-(3-fluoro-4-(trifluoromethyl)phenyl)-1,3,4-thiadiazole (f-6)

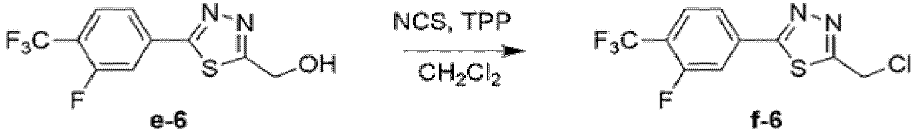

Compound f-6 (0.234 g, 79% yield) was obtained in the same manner as in Step 5 of Example 1, except that Compound e-6 was used instead of Compound e-1.

### Step 6: Preparation of 4-(((5-(3-fluoro-4-(trifluoromethyl)phenyl)-1,3,4-thiadiazol-2-yl)methyl)thio)-2-methylphenol (g-6)

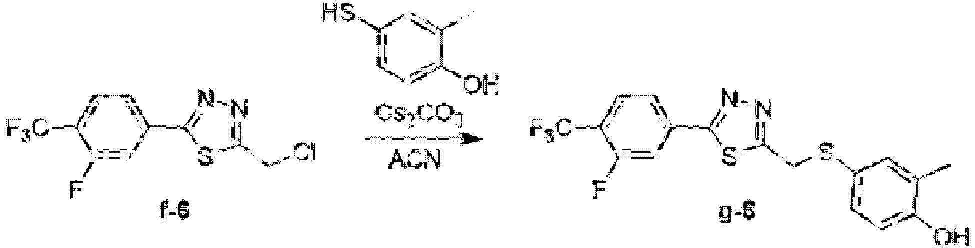

Compound g-6 (0.401 g, 97% yield) was prepared in the same manner as in Step 6 of Example 1, except that Compound f-6 was used instead of Compound f-1.

### Step 7: Preparation of methyl 2-(4-(((5-(3-fluoro-4-(trifluoromethyl)phenyl)-1,3,4-thiadiazol-2-yl)methyl)thio)-2-methylphenoxy)acetate (h-6)

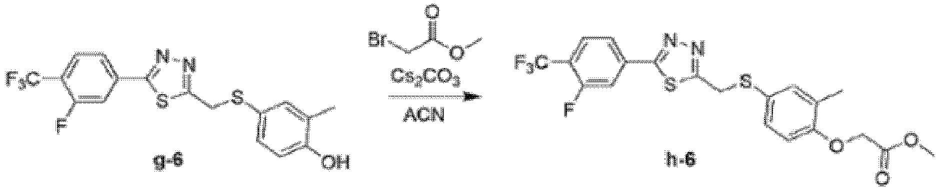

Compound h-6 (0.421 g, 89% yield) was obtained in the same manner as in Step 7 of Example 1, except that Compound g-6 was used instead of Compound g-1.

### Step 8: Preparation of 2-(4-(((5-(3-fluoro-4-(trifluoromethyl)phenyl)-1,3,4-thiadiazol-2-yl)methyl)thio)-2-methylphenoxy)acetic acid (19)

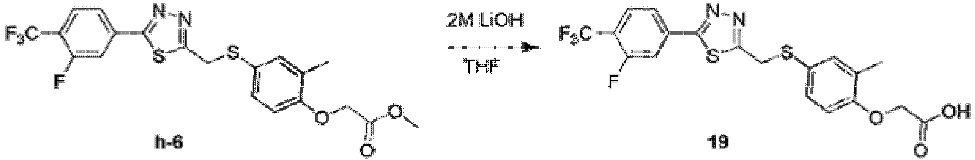

Compound 19 (0.264 g, 60% yield) was obtained in the same manner as in Step 8 of Example 1, except that Compound h-6 was used instead of Compound h-1.

¹H NMR (CD₃OD, 400 MHz) δ 7.97 (d, *J =* 11.2 Hz, 1H), 7.92 - 7.85 (m, 2H), 7.29 (s, 1H), 7.24 (d, *J =* 8.5 Hz, 1H), 6.77 (d, *J =* 8.4 Hz, 1H), 4.67 (s, 2H), 4.52 (s, 2H), 2.23 (s, 3H). ¹³C NMR (DMSO-d₆, 100 MHz) δ 171.74, 170.62, 166.53, 159.52 (d, JCF = 253.5 Hz), 156.42, 136.22, 134.06, 130.59, 129.04, 127.69, 124.63, 123.84, 122.76 (q, JCF = 271.4 Hz), 116.32, 112.57, 65.46, 33.32, 16.38.

### <Example 20> Preparation of 2-(4-(((5-(3-fluoro-4-(trifluoromethyl)phenyl)-1,3,4-thiadiazol-2-yl)methyl)thio)-2-methylphenoxy)propanoic acid

### Step 1: Preparation of ethyl 2-(4-(((5-(3-fluoro-4-(trifluoromethyl)phenyl)-1,3,4-thiadiazol-2-yl)methyl)thio)-2-methylphenoxy)propanoate (i-6)

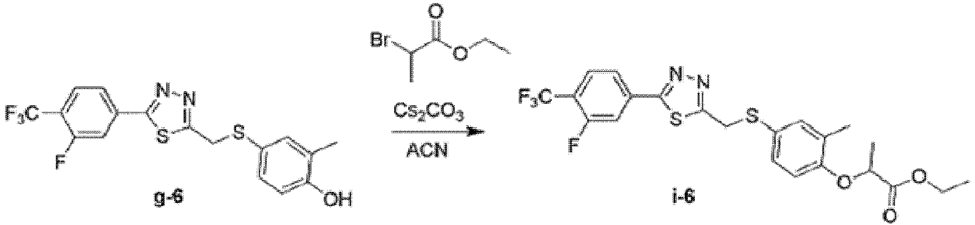

Compound i-6 (0.360 g, 72%) was obtained in the same manner as in Step 7 of Example 1, except that Compound g-6 was used instead of Compound g-1, and the compound ethyl 2-bromopropanoate was used instead of the compound methyl 2-bromoacetate.

### Step 2: Preparation of 2-(4-(((5-(3-fluoro-4-(trifluoromethyl)phenyl)-1,3,4-thiadiazol-2-yl)methyl)thio)-2-methylphenoxy)propanoic acid (20)

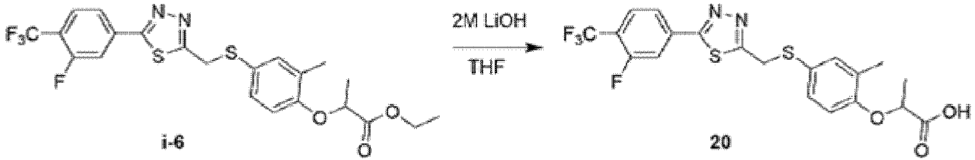

Compound 20 (0.300 g, 66% yield) was obtained in the same manner as in Step 8 of Example 1, except that Compound i-6 was used instead of Compound h-1.

¹H NMR (CDCl₃, 400 MHz) δ7.81 - 7.68 (m, 3H), 7.22 (s, 1H), 7.15 (dd, *J =* 8.5, 1.9 Hz, 1H), 6.62 (d, *J =* 8.5 Hz, 1H), 4.73 (q, *J =* 6.8 Hz, 1H), 4.42 (s, 2H), 2.21 (s, 3H), 1.65 (d, *J =* 6.8 Hz, 3H). ¹³C NMR (CDCl₃, 100 MHz) δ176.22, 171.02, 166.72, 159.89 (d, JCF = 254.5 Hz), 155.96, 135.47, 134.94, 130.83, 128.99, 128.16, 124.24, 123.43, 119.53(q, JCF = 246.2 Hz), 115.87, 112.50, 72.39, 34.40, 18.48, 16.23.

### <Example 21> Preparation of 2-(4-(((5-(3-fluoro-4-(trifluoromethyl)phenyl)-1,3,4-thiadiazol-2-yl)methyl)thio)-2-methylphenoxy)-2-methylpropanoic acid

### Step 1: Preparation of ethyl 2-(4-(((5-(3-fluoro-4-(trifluoromethyl)phenyl)-1,3,4-thiadiazol-2-yl)methyl)thio)-2-methylphenoxy)-2-methylpropanoate (j-6)

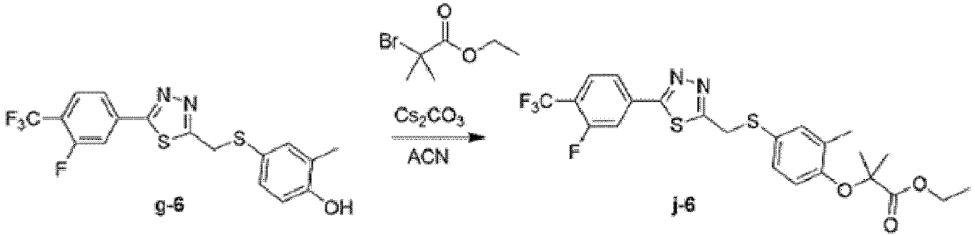

Compound j-6 (52 mg, 10% yield ) was obtained in the same manner as in Step 7 of Example 1, except that Compound g-6 was used instead of Compound g-1, and ethyl 2-bromo-2-methylpropanoate was used instead of methyl 2-bromoacetate.

### Step 2: Preparation of 2-(4-(((5-(3-fluoro-4-(trifluoromethoxy)phenyl)-1,3,4-thiadiazol-2-yl)methyl)thio)-2-methylphenoxy)-2-methylpropanoic acid (21)

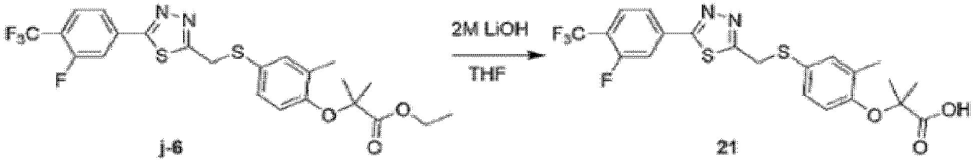

Compound 21 (80 mg, 17% yield) was obtained in the same manner as in Step 8 of Example 1, except that Compound j-6 was used instead of Compound h-1.

¹H NMR (CDCl₃, 400 MHz) δ7.81 - 7.69 (m, 3H), 7.21 (s, 1H), 7.09 (d, *J =* 8.2 Hz, 1H), 6.69 (d, *J =* 8.6 Hz, 1H), 2.17 (s, 3H), 0.88 (s, 6H).

### <Example 22> Preparation of 2-(2-methyl-4-(((5-(p-tolyl)-1,3,4-thiadiazol-2-yl)methyl)thio)phenoxy)acetic acid

### Step 1: Preparation of 4-methylbenzohydrazide (b-7)

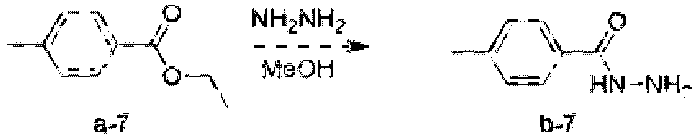

Compound b-7 (0.78 g, 85% yield) was obtained in the same manner as in Step 1 of Example 1, except that Compound a-7 was used instead of Compound a-1.

### Step 2: Preparation of ethyl 2-(2-(4-methylbenzoyl)hydrazinyl)-2-oxoacetate (c-7)

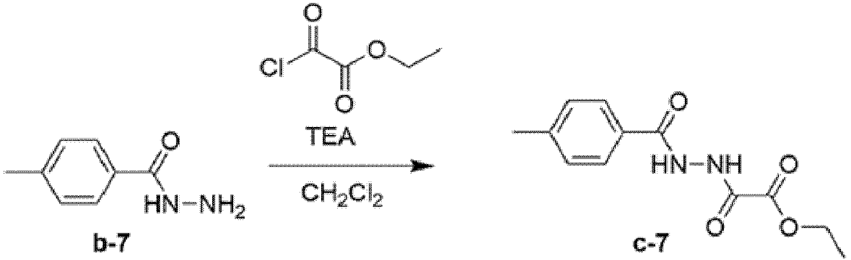

Compound c-7 (1.25 g, 96% yield) was obtained in the same manner as in Step 2 of Example 1, except that Compound b-7 was used instead of Compound b-1.

### Step 3: Preparation of ethyl 5-(p-tolyl)-1,3,4-thiadiazole-2-carboxylate (d-7)

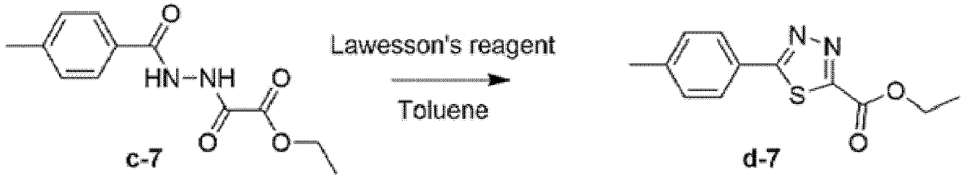

Compound d-7 (0.840 g, 67.7% yield) was obtained in the same manner as in Step 3 of Example 1, except that Compound c-7 was used instead of Compound c-1.

### Step 4: Preparation of (5-(p-tolyl)-1,3,4-thiadiazol-2-yl)methanol (e-7)

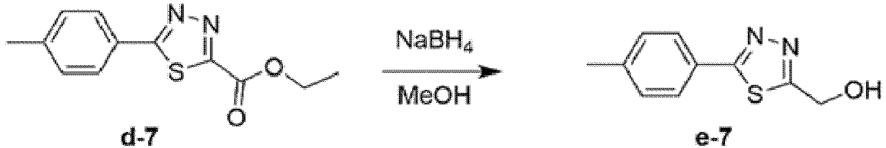

Compound e-7 (0.690 g, 99% yield) was obtained in the same manner as in Step 4 of Example 1, except that Compound d-7 was used instead of Compound d-1.

### Step 5: Preparation of 2-(chloromethyl)-5-(p-tolyl)-1,3,4-thiadiazole (f-7)

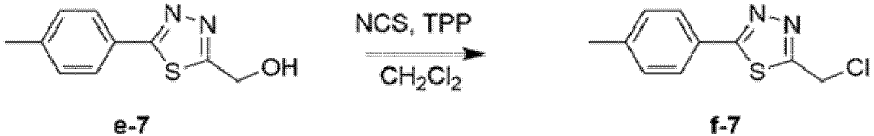

Compound f-7 (0.359 g, 47.8% yield) was obtained in the same manner as in Step 5 of Example 1, except that Compound e-7 was used instead of Compound e-1.

### Step 6: Preparation of 2-methyl-4-(((5-(p-tolyl)-1,3,4-thiadiazol-2-yl)methyl)thio)phenol (g-7)

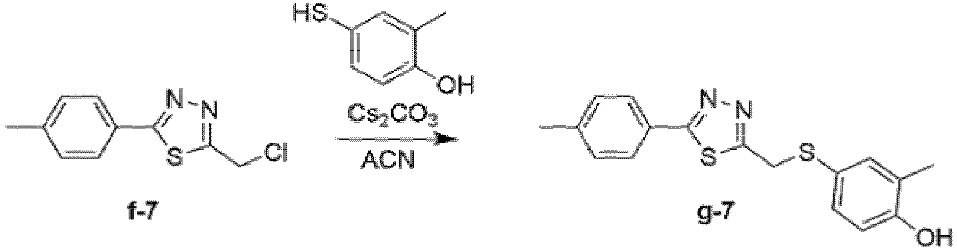

Compound g-7 (0.560 g, 97% yield) was obtained in the same manner as in Step 6 of Example 1, except that Compound f-7 was used instead of Compound f-1.

### Step 7: Preparation of methyl 2-(2-methyl-4-(((5-(p-tolyl)-1,3,4-thiadiazol-2-yl)methyl)thio)phenoxy)acetate (h-7)

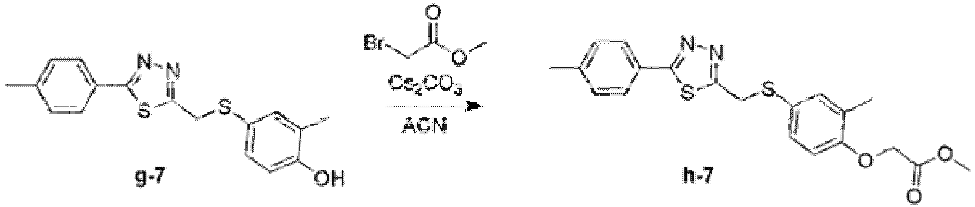

Compound h-7 (70 mg, 97% yield) was obtained in the same manner as in Step 7 of Example 1, except that Compound g-7 was used instead of Compound g-1.

### Step 8: Preparation of 2-(2-methyl-4-(((5-(p-tolyl)-1,3,4-thiadiazol-2-yl)methyl)thio)phenoxy)acetic acid (22)

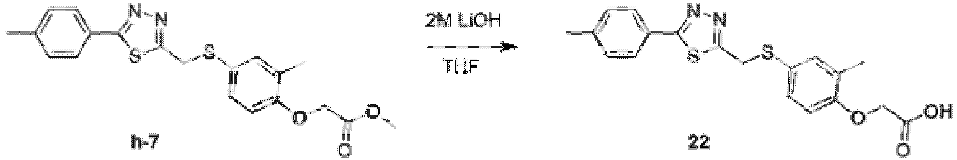

Compound 22 (34 mg, 96% yield) was obtained in the same manner as in Step 8 of Example 1, except that Compound h-7 was used instead of Compound h-1.

ESI LC/MS: m/z calcd. for C₁₉H₁₉N₂O₃S₂ [M+H]⁺: 387.09; found 386.8. ¹H NMR (DMSO, 400MHz) δ 7.80 (d, *J =* 7.96 Hz, 2H), 7.34 (d, *J =* 7.84 Hz, 2H), 7.24 (s, 1H), 7.19 (d, *J =* 8.28 Hz, 1H), 6.73 (d, *J =* 8.56 Hz, 1H), 4.57 (s, 2H), 4.47 (s, 2H), 2.36 (s, 3H), 2.12 (s, 3H).

### <Example 23> Preparation of 2-(2-methyl-4-(((5-(p-tolyl)-1,3,4-thiadiazol-2-yl)methyl)thio)phenoxy)propanoic acid

### Step 1: Preparation of ethyl 2-(2-methyl-4-(((5-(p-tolyl)-1,3,4-thiadiazol-2-yl)methyl)thio)phenoxy)propanoate (i-7)

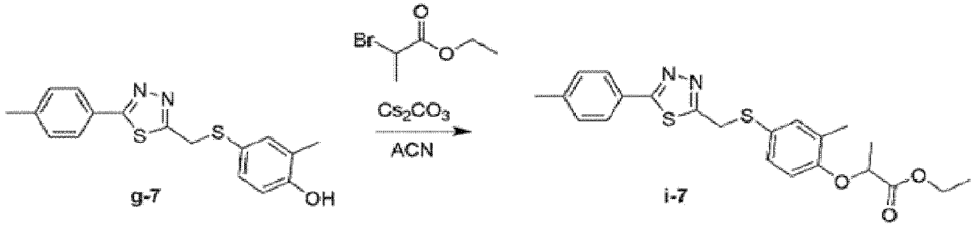

Compound i-7 (0.100 g, 97% yield) was obtained in the same manner as in Step 7 of Example 1, except that Compound g-7 was used instead of Compound g-1, and ethyl 2-bromopropanoate was used instead of methyl 2-bromoacetate.

### Step 2: Preparation of 2-(2-methyl-4-(((5-(p-tolyl)-1,3,4-thiadiazol-2-yl)methyl)thio)phenoxy)propanoic acid (23)

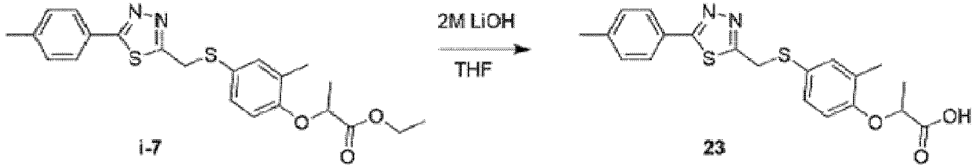

Compound 23 (34 mg, 35% yield) was obtained in the same manner as in Step 8 of Example 1, except that Compound i-7 was used instead of Compound h-1.

ESI LC/MS: m/z calcd. for C₂₀H₂₁N₂O₃S₂ [M+H]⁺: 401.10; found 401.1. ¹H NMR (CD₃OD, 400MHz) δ 7.78 (d, *J =* 7.84 Hz, 2H), 7.33 (d, *J =* 7.76 Hz, 2H), 7.25 (s, 1H), 7.21 (d, *J =* 8.44 Hz, 1H), 6.72 (d, *J =* 8.40 Hz, 1H), 4.81 (m, 1H), 4.44 (s, 1H), 2.40 (s, 3H), 2.21 (s, 3H), 1.63 (d, *J =* 6.76 Hz, 3H). ¹³C NMR (CD₃OD, 100 MHz) δ15.00, 17.56, 20.07, 33.64, 60.71, 72.35, 112.03, 123.76, 126.84, 127.65(2C), 128.29, 129.64 (2C), 131.19, 134.87, 141.99, 156.37, 169.57, 170.04.

### <Example 24> Preparation of 2-methyl-2-(2-methyl-4-(((5-(p-tolyl)-1,3,4-thiadiazol-2-yl)methyl)thio)phenoxy)propanoic acid

### Step 1: Preparation of ethyl 2-methyl-2-(2-methyl-4-(((5-(p-tolyl)-1,3,4-thiadiazol-2-yl)methyl)thio)phenoxy)propanoate (j-7)

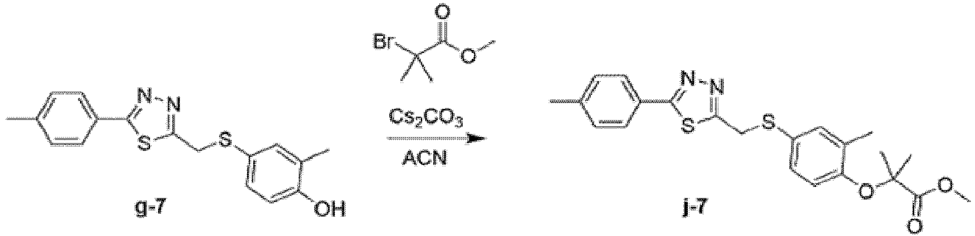

Compound j-7 (94 mg, 97% yield) was obtained in the same manner as in Step 7 of Example 1, except that Compound g-7 was used instead of Compound g-1,and ethyl 2-bromo-2-methylpropanoate was used instead of methyl 2-bromoacetate.

### Step 2: Preparation of 2-methyl-2-(2-methyl-4-(((5-(p-tolyl)-1,3,4-thiadiazol-2-yl)methyl)thio)phenoxy)propanoic acid (24)

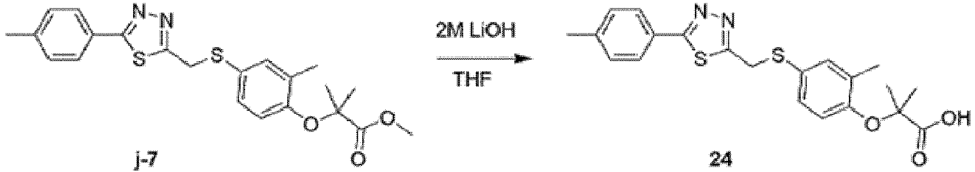

Compound 24 (2 mg, 3% yield) was obtained in the same manner as in Step 8 of Example 1, except that Compound j-7 was used instead of Compound h-1.

¹H NMR (CD₃OD, 400 MHz) δ 7.76 (d, *J =* 8.20 Hz, 2H), 7.32 (d, *J =* 8.04 Hz, 2H), 7.26 (s, 1H), 7.17 (dd, *J =* 2.32 Hz, 6.20 Hz 1H), 6.72 (d, *J =* 8.48 Hz, 1H), 4.45 (s, 2H), 2.39 (s, 3H), 2.16 (s, 3H), 1.57 (s, 6H). ¹³C NMR (CD₃OD, 100 MHz) δ15.42, 20.08, 24.35(2C), 78.82, 116.68, 124.53, 126.82, 127.31 (2C), 129.64 (2C), 130.34, 134.79, 142.01, 154.16, 169.46, 170.05, 176.24.

### <Example 25? Preparation of 2-(2-methyl-4-(((5-(4-(trifluoromethoxy)phenyl)-1,3,4-thiadiazol-2-yl)methyl)thio)phenoxy)acetic acid

### Step 1: Preparation of 4-(trifluoromethyl)benzohydrazide (b-8)

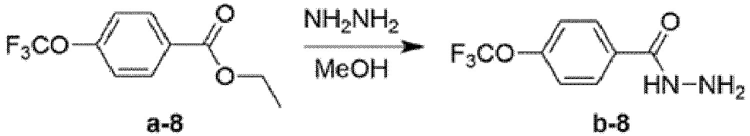

Compound b-8 (1.16 g, 53%) was obtained in the same manner as in Step 1 of Example 1, except that Compound a-8 was used instead of Compound a-1.

### Step 2: Preparation of ethyl 2-(2-(4-(fluoroxy)benzoyl)hydrazinyl)-2-oxoacetate (c-8)

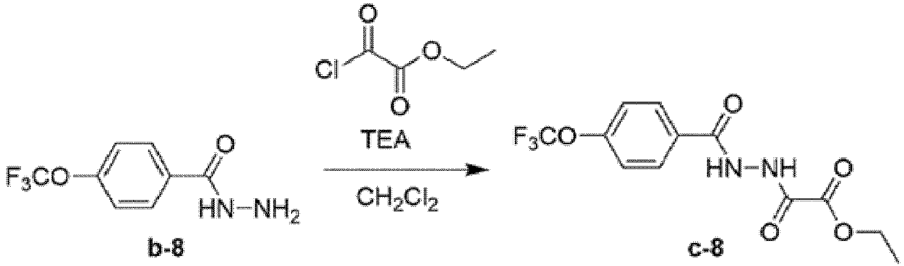

Compound c-8 (0.275 g, 86% yield) was obtained in the same manner as in Step 2 of Example 1, except that Compound b-8 was used instead of Compound b-1.

### Step 3: Preparation of ethyl 5-(4-(trifluoromethoxy)phenyl)-1,3,4-thiadiazole-2-carboxylate (d-8)

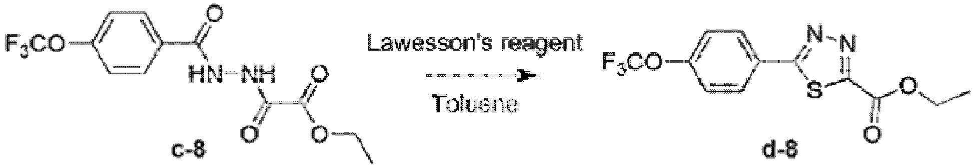

Compound d-8 (0.352 g, 85% yield) was obtained in the same manner as in Step 3 of Example 1, except that Compound c-8 was used instead of Compound c-1.

### Step 4: Preparation of (5-(4-(trifluoromethoxy)phenyl)-1,3,4-thiadiazol-2-yl)methanol (e-8)

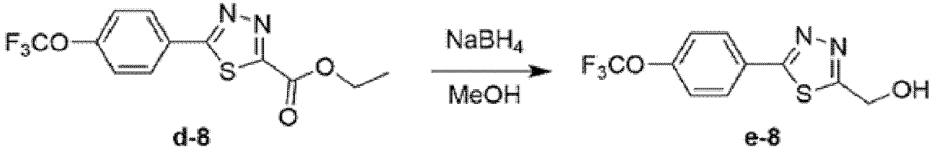

Compound e-8 (0.249 g, 90% yield) was obtained in the same manner as in Step 4 of Example 1, except that Compound d-8 was used instead of Compound d-1.

### Step 5: Preparation of 2-(chloromethyl)-5-(4-(trifluoromethoxy)phenyl)-1,3,4-thiadiazole (f-8)

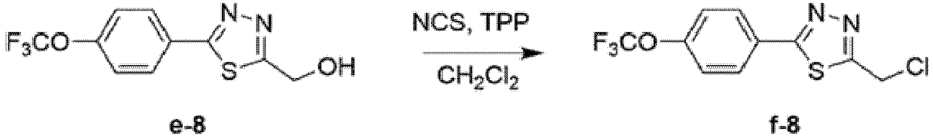

Compound f-8 (0.206 g, 70% yield) was obtained in the same manner as in Step 5 of Example 1, except that Compound e-8 was used instead of Compound e-1.

### Step 6: Preparation of 2-methyl-4-(((5-(4-(trifluoromethoxy)phenyl)-1,3,4-thiadiazol-2-yl)methyl)thio)phenol (g-8)

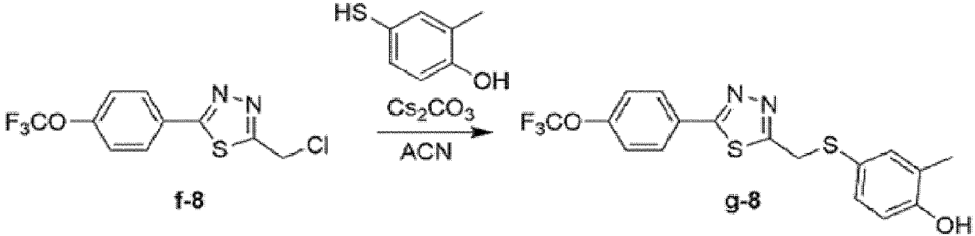

Compound g-8 (0.398 g, 97% yield) was obtained in the same manner as in Step 6 of Example 1, except that Compound f-8 was used instead of Compound f-1.

### Step 7: Preparation of methyl 2-(2-methyl-4-(((5-(4-(trifluoromethoxy)phenyl)-1,3,4-thiadiazol-2-yl)methyl)thio)phenoxy)acetate (h-8)

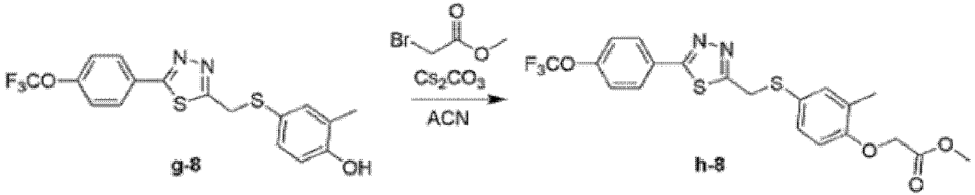

Compound h-8 (0.409 g, 87% yield) was obtained in the same manner as in Step 7 of Example 1, except that Compound g-8 was used instead of Compound g-1.

### Step 8: Preparation of 2-(2-methyl-4-(((5-(4-(trifluoromethoxy)phenyl)-1,3,4-thiadiazol-2-yl)methyl)thio)phenoxy)acetic acid (25)

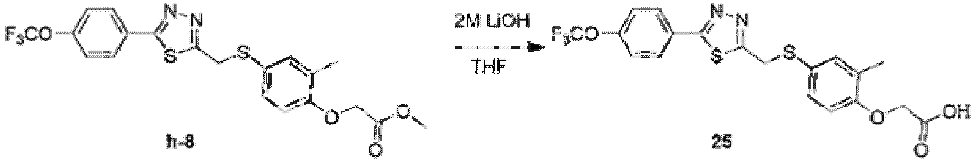

Compound 25 (0.393 g, 86% yield) was obtained in the same manner as in Step 8 of Example 1, except that Compound h-8 was used instead of Compound h-1,

¹H NMR (DMSO-d₆, 400 MHz) δ8.09 - 8.05 (m, 2H), 7.54 (d, *J =* 8.1 Hz, 2H), 7.29 (d, *J=* 1.8 Hz, 1H), 7.21 (dd, *J = 8.5,* 2.3 Hz, 1H), 6.78 (d, *J =* 8.6 Hz, 1H), 4.69 (s, 2H), 4.64 (s, 2H), 2.14 (s, 3H); ¹³C NMR (DMSO-d₆, 100 MHz) δ170.55, 170.39, 167.64, 156.26, 150.55, 134.09, 130.60, 130.21, 129.17, 127.68, 124.06, 122.90 (q, JCF = 239.8 Hz), 122.30, 112.53, 65.17, 33.30, 16.38.

### <Example 26> Preparation of 2-(2-methyl-4-(((5-(4-(trifluoromethoxy)phenyl)-1,3,4-thiadiazol-2-yl)methyl)thio)phenoxy)propanoic acid

### Step 1: Preparation of ethyl 2-(2-methyl-4-(((5-(4-(trifluoromethoxy)phenyl)-1,3,4-thiadiazol-2-yl)methyl)thio)phenoxy)propanoate (i-8)

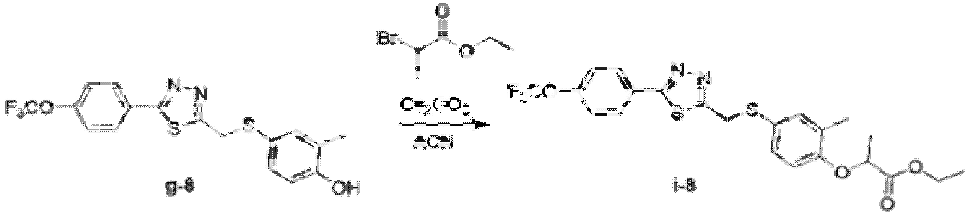

Compound i-8 (0.379 g, 76% yield) was obtained in the same manner as in Step 7 of Example 1, except that Compound g-8 was used instead of Compound g-1, and the compound ethyl 2-bromopropanoate was used instead of methyl 2-bromoacetate.

### Step 2: Preparation of 2-(2-methyl-4-(((5-(4-(trifluoromethoxy)phenyl)-1,3,4-thiadiazol-2-yl)methyl)thio)phenoxy)propanoic acid (26)

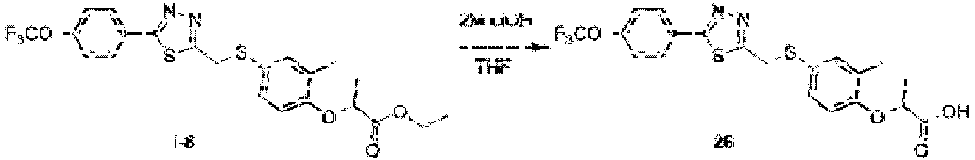

Compound 26 (0.409 g, 87% yield) was obtained in the same manner as in Step 8 of Example 1, except that Compound i-8 was used instead of Compound h-1.

¹H NMR (DMSO-d6, 400 MHz) δ8.09 - 8.05 (m, 2H), 7.54 (d, *J =* 8.1 Hz, 2H), 7.29 (d, *J =* 1.8 Hz, 1H), 7.21 (dd, *J =* 8.5, 2.3 Hz, 1H), 6.72 (d, *J =* 8.6 Hz, 1H), 4.80 (q, *J =* 6.7 Hz, 1H), 4.63 (s, 2H), 2.14 (s, 3H), 1.50 (d, *J =* 6.8 Hz, 3H). ¹³C NMR (DMSO-d6, 100 MHz) δ173.44, 170.38, 167.64, 156.03, 150.57, 134.13, 130.56, 130.21, 129.17, 127.92, 124.03, 122.30, 120.42 (q, JCF = 255.7 Hz), 112.98, 72.26, 33.29, 18.79, 16.39.

### <Example 27> Preparation of 2-methyl-2-(2-methyl-4-(((5-(4-(trifluoromethoxy)phenyl)-1,3,4-thiadiazol-2-yl)methyl)thio)phenoxy)propanoic acid

### Step 1: Preparation of ethyl 2-methyl-2-(2-methyl-4-(((5-(4-(trifluoromethoxy)phenyl)-1,3,4-thiadiazol-2-yl)methyl)thio)phenoxy)propanoate (j-8)

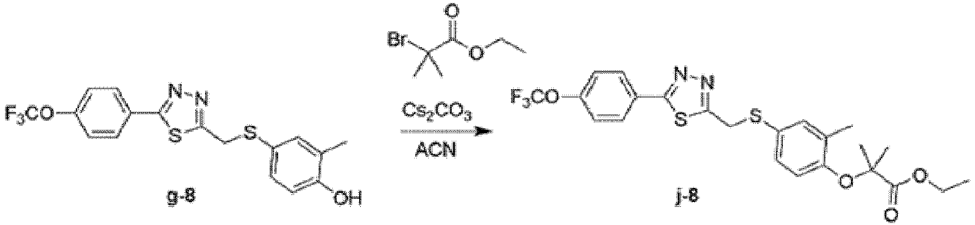

Compound j-8 (0.179 g, 35% yield) was obtained in the same manner as in Step 7 of Example 1, except that Compound g-8 was used instead of Compound g-1, and the compound ethyl 2-bromo-2-methylpropanoate was used instead of methyl 2-bromoacetate.

### Step 2: Preparation of 2-methyl-2-(2-methyl-4-(((5-(4-(trifluoromethoxy)methyl)-1,3,4-thiadiazol-2-yl)methyl)thio)phenoxy)propanoic acid (27)

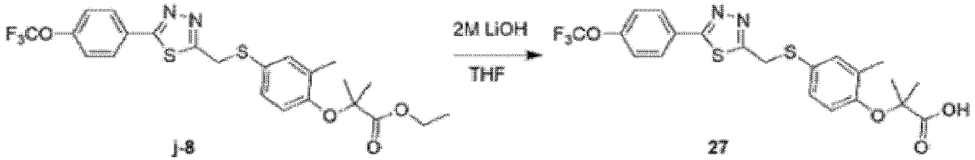

Compound 27 (0.170 g, 35% yield) was obtained in the same manner as in Step 8 of Example 1, except that Compound j-8 was used instead of Compound h-1.

ESI LC/MS: m/z calcd. for C₂₁H₂₀F₃N₂O₄S₂ [M+H]⁺: 485.08; found 485. ¹H NMR (CDCl₃, 400 MHz) δ7.92 (d, *J =* 8.2 Hz, 2H), 7.29 (d, *J =* 8.2 Hz, 2H), 7.18 (s, 1H), 7.09 (s, 1H), 6.68 (s, 1H), 4.41 (s, 2H), 2.14 (s, 3H), 1.25 (s, 6H). ¹³C NMR (CDCl₃, 100 MHz) δ169.86, 168.17, 160.90, 151.16, 142.81, 134.57, 134.52, 129.89, 129.47, 128.46, 121.60, 121.38, 119.03, 34.13, 29.72, 25.19, 16.74.

The compounds of Examples 1 to 27 are summarized in Tables 1 and 2 below.

**TABLE 1**

| No. | Chemical Formula | No. | Chemical Formula |
|---|---|---|---|
| 1 | 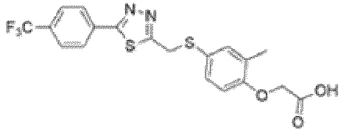 | 2 | 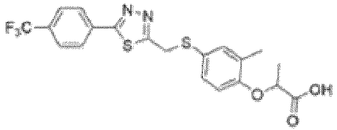 |
| 3 | 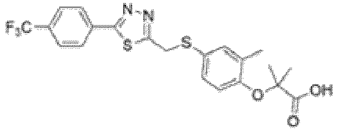 | 4 | 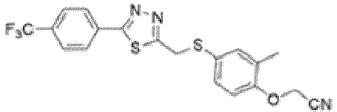 |
| 5 | 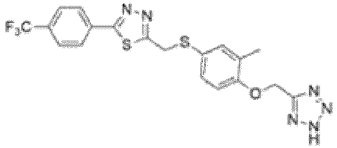 | 6 | 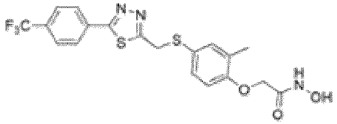 |
| 7 | 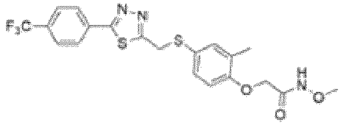 | 8 | 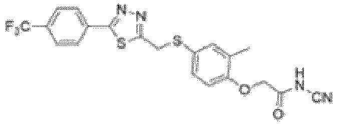 |
| 9 | 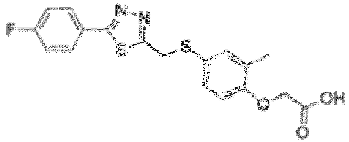 | 10 | 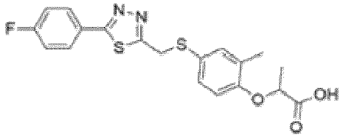 |
| 11 | 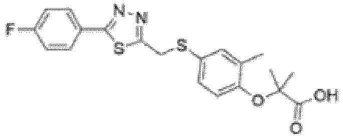 | 12 | 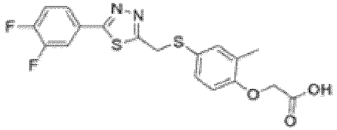 |
| 13 | 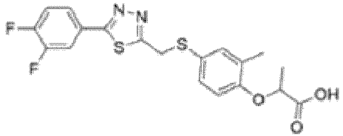 | 14 | 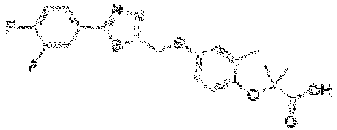 |

**TABLE 2**

| No. | Chemical Formula | No. | Chemical Formula |
|---|---|---|---|
| 15 | 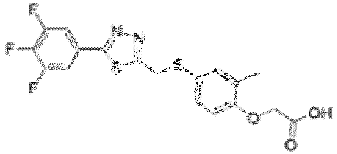 | 16 | 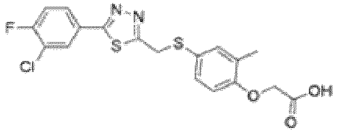 |
| 17 | 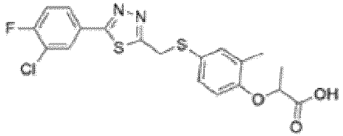 | 18 | 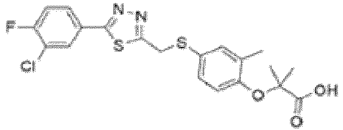 |
| 19 | 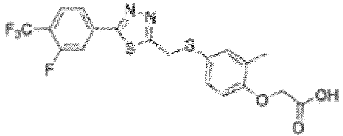 | 20 | 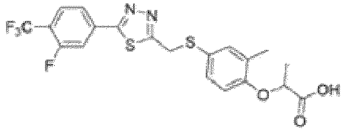 |
| 21 | 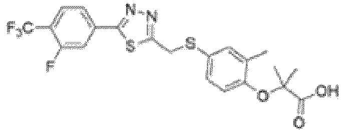 | 22 | 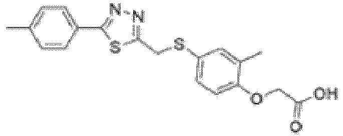 |
| 23 | 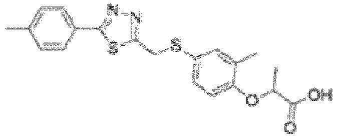 | 24 | 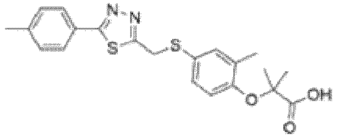 |
| 25 | 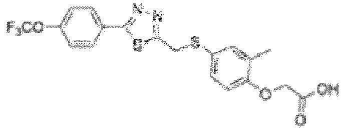 | 26 | 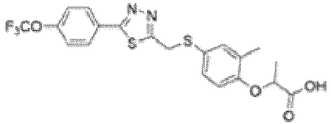 |
| 27 | 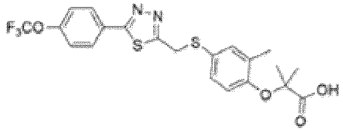 | | |

### <Experiment Method>

### 1. Animals

Germ-free, 6-week-old immunocompetent C57BL/6 mice were obtained from SLC, Inc (Shizuoka, Japan). All animal experimental procedures were conducted in strict accordance with the appropriate institutional guidelines for animal research. The protocol was approved by the Animal Experiment Ethics Committee of Kyungpook National University (approval number: KNU 2012-43).

### 2. Cell culture

RAW 264.7 cells, which are macrophages, were purchased from the Korea Cell Line Bank and used, and were cultured in DMEM with 100 mg/mL inactivated fetal bovine serum and 10 mg/mL penicillin-streptomycin added as culture medium at 37°C and 5% CO₂ condition. All cells in the experimental process were subcultured when grown to a density of about 80% to 90%, and only cells that did not exceed 20 passages were used.

### 3. Cytotoxicity measurement

To evaluate the cytotoxicity of the sample, cell proliferation was tested using a cell counting kit [CCK-8; Dojindo Laboratories (Tokyo, Japan)]. RAW 264.7 cell 1 × 10⁴ cells/mL were dispensed into a well plate, and after pre-culturing for 20 hours, 1 µg/mL of LPS and example compounds were added at different concentrations and cultured for 24 hours in an incubator at 37°C and 5% CO₂. After culturing, 10 µl CCK-8 reagent was added and recultured for 1 to 2 hours, and the absorbance was measured. The absorbance was measured at 450 nm using a microplate reader [BMG Labtech (located in Offenburg, Germany)].

### 4. Nitric Oxide (NO) production measurement

The concentration of NO was measured using the nitrite concentration in the culture medium with the Griess reagent system (Promega). Raw264.7 cells were adjusted to 2.5 × 10⁵ cells/mL using DMEM medium, inoculated into 24-well plates, and pre-cultured for 20 hours in a 5% CO₂ incubator. The cells were treated with 1 µg/mL of LPS and various concentrations of the example compounds and recultured for 24 hours. After obtaining the supernatant of the culture medium, the NO concentration was measured using the Griess reagent system according to the protocol provided by the manufacturer. The absorbance was measured at 540 nm using a microplate reader [BMG Labtech (located in Offenburg, Germany)]. The concentration of NO in the cell culture medium was calculated by comparing it with a standard curve for each concentration of of sodium nitrite (NaNO₂).

### 5. Inflammatory-related cytokine secretion measurement

The supernatant was dispensed using the same method as used for measuring NO. High amounts of TNF-α and IL-6 in the supernatant were quantified using the multiplex bead-based immunoassay (BDTM Cytometric Bead Array) system and flow cytometry (BD Biosciences).

### 6. Luciferase assay

Raw264.7/NF-kb-luc cells were treated with 1 mg/mL LPS and various concentrations of compounds using the same method as used for measuring NO. 16 hours later, after adding the D-luciferin solution to the cells, luminescence images were obtained using the IVIS Lumina III. Quantification of the luminescence images was performed using Living Imaging software.

### 7. Western blot

The cells were washed twice with cold PBS and lysed with RIPA buffer (Roche) containing a complete protease inhibitor cocktail. Protein concentration was quantified using the BCA protein assay kit (Pierce, IL, USA), and 30 µL of lysate was separated by 10% SDS-PAGE. The separated proteins were transferred to a polyvinylidene difluoride (PVDF) membrane (Biorad, CA, USA) at 70 mA for 1 hour and 30 minutes, then blocked with tris-buffered saline (TBST; pH 7.5) containing 50 mg/mL skim milk at room temperature for 2 hours. As antibodies to examine the expression levels of iNOS and pIKKαβ, anti-iNOS and pIKKαβ were used, diluted 1: 1000 and reacted at room temperature for 2 hours, followed by washing three times with TBST. As secondary antibodies, horseradish peroxidase (HRP)-conjugated anti-mouse IgG and anti-rabbit IgG were diluted 1:2000 and reacted at room temperature for 1 hour, followed by washing three times with TBST. Peroxidase activity was detected using ECL-Plus according to the manufacturer's protocol.

### 8. Macrophage image analysis

2 mL of thioglycollate medium (BD, USA) was injected into the abdominal cavity of a C57Bl/6 mouse, and three days later, the mouse was cervically dislocated, and 8 mL of Dulbecco's modified Eagle's medium (DMEM) was injected into the abdominal cavity to collect cells. After centrifugation, the cells were suspended in DMEM containing 10% fetal bovine serum (FBS) and 1% penicillin-streptomycin and cultured in a 5% CO₂ incubator at 37°C for 2 hours. Non-adherent cells were removed, and the remaining cells were used for experiments.

The acute anti-inflammatory activity of the example compound was evaluated using a macrophage tracking technique with fluorescence imaging. Macrophages extracted from the mouse abdominal cavity were labeled with Vybrant^{™} DiD Cell-Labeling Solution and then administered to the mouse via intravenous injection. The next day, 50 mg/kg of the example compound was orally administered, and 1 hour later, a 0.1% Carrageenan (CG) solution or PBS was subcutaneously injected into the right or left sole of the mouse. Fluorescent images were obtained using IVIS Lumina III at the designated time.

### 9. Statistical analysis

All data were expressed as mean ± standard deviation (SD) from at least three independent tests, and statistical significance of differences was determined by unpaired Student's test using GrahPad Prism 5. P values less than 0.05 were considered statistically significant.

### <Experimental Example 1> Enzyme activity (EC₅₀) analysis

In order to confirm the PPARδ activation effect of the example compounds, the following experiment was performed. First, the example compounds were diluted in DMSO to 100X based on the final concentration, then 1/50 was diluted in assay buffer, and 10 µL of each was added to a 384 well plate. Afterwards, GST-conjugated PPARδ ligand-binding domain (LBD) was added to a final concentration of 5 nM, and fluorescien-conjugated C33 coactivator peptide and Tb-α-GST antibody were added to a final concentration of 100 nM and 10 nM, respectively. The final volume of each test system was set to 20 µL, and the test for each concentration was repeated twice, and the binding activity after reaction was measured by TR-FRET method. In other words, after excitation at 340 nm and measurement of emission values at 495 nm and 520 nm, the results were analyzed using the measured value at 490 nm/measured at 520 nm, and the analysis program Prism 6 was used to calculate the EC₅₀ value. The results are summarized in Table 2 below.

The principles of this test method are as follows. GST antibody binds to GST fused to the PPARδ LBD, thereby maintaining its inactive state, and binding of the agonist induces a structural change in the LBD protein. At this time, fluorescence energy is emitted as the fluorescence energy-donor bound to the GST antibody transfers energy to the fluorescence energy-acceptor (fluorescine) bound to the PPARδ substrate (C33 peptide), and by measuring this value, the degree of activation of PPARδ can be measured.

Meanwhile, the previously known PPARδ agonist compound GW501516 was used as a test material, and as a result of the experiment, the EC₅₀ value in the case of GW501516 was 15.5 nM.

**TABLE 3**

| No. | Enzymatic activity (EC₅₀, µM) | No. | Enzymatic activity (EC₅₀, µM) |
|---|---|---|---|
| 1 | 0.0399 | 2 | 0.42 |
| 3 | 0.374 | 8 | 0.504 |
| 9 | 2.437 | 12 | 0.663 |
| 13 | 6.157 | 14 | 2.099 |
| 15 | 4.014 | 16 | 1.349 |
| 18 | 2.21 | 19 | 0.289 |
| 20 | 2.356 | 21 | 2.66 |
| 22 | 0.574 | 23 | 5.697 |
| 24 | 1.252 | 25 | 0.042 |
| 26 | 1.243 | 27 | 0.595 |

<Experimental Example 2> Cytotoxicity analysis

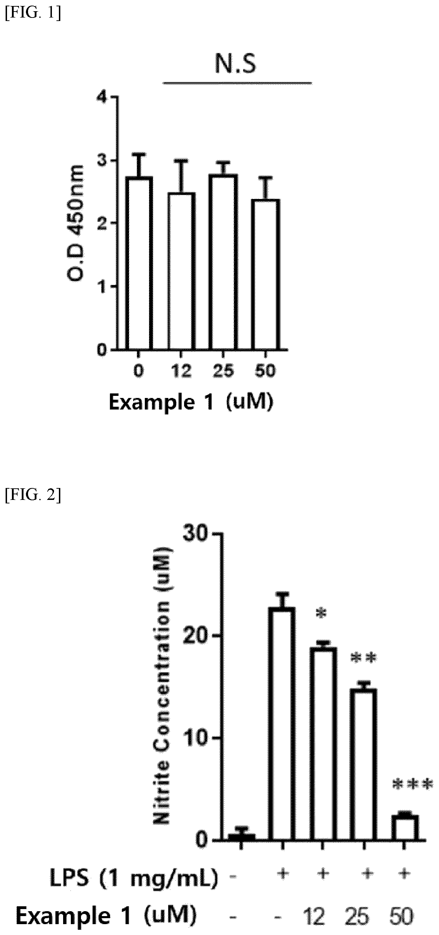

In order to evaluate the cytotoxicity of the compound of Example 1, Raw264.7 cells, a macrophage cell line, were treated with the compound of Example 1 at concentrations of 0, 12 µM, 25 µM, and 50 µM, respectively, and then CCK8 assay was performed according to <Experiment Method> 3 above. CCK8 assay results showed no cytotoxicity at all concentrations (FIG. 1). Specifically, cell proliferation was similar to that of the vehicle group even at a concentration of up to 50 µM, and no significance was detected.

### <Experimental Example 3> Nitric Oxide (NO) production amount analysis

NO is produced from L-arginine by nitric oxide synthase (NOS), which exists in various tissues. There are three types of NOS, which are neuronal NOS, endothelial NOS, and inducible NOS (iNOS), and among these, iNOS, also called NOS-2, is expressed in various cells, including macrophages, by inflammatory stimuli such as bacterial endotoxin and various inflammatory cytokines during bacterial infection, and once iNOS is expressed, a large amount of NO is produced for a long period of time and participates in cytotoxicity during inflammation. In order to analyze the NO production amount by the compound of Example 1, the following experiment was performed.

1 mg/mL lipopolysaccharide (LPS) and the compounds of Example 1 at concentrations of 0, 12 µM, 25 µM, and 50 µM were added to Raw264.7 cells, cultured for 24 hours, and changes in NO concentration in the upper layer of the culture were analyzed.

As a result of the analysis, the concentration of NO secreted from LPS-stimulated Raw264.7 decreased in a concentration-dependent manner of Example Compound 1, and specifically, the NO concentrations produced in LPS-stimulated Raw26.7 + 0 µM, LPS-stimulated Raw26.7 + 12.5 µM, LPS-stimulated Raw26.7 + 25 µM and LPS-stimulated Raw26.7 + 50 µM, respectively, were 22.8±1.3 µM, 18.9±0.3 µM, 14.8±0.54 µM and 2.41±0.24 µM (FIG. 2).

### <Experimental Example 4> Analysis of iNOS protein expression inhibitory effect (Western blot)

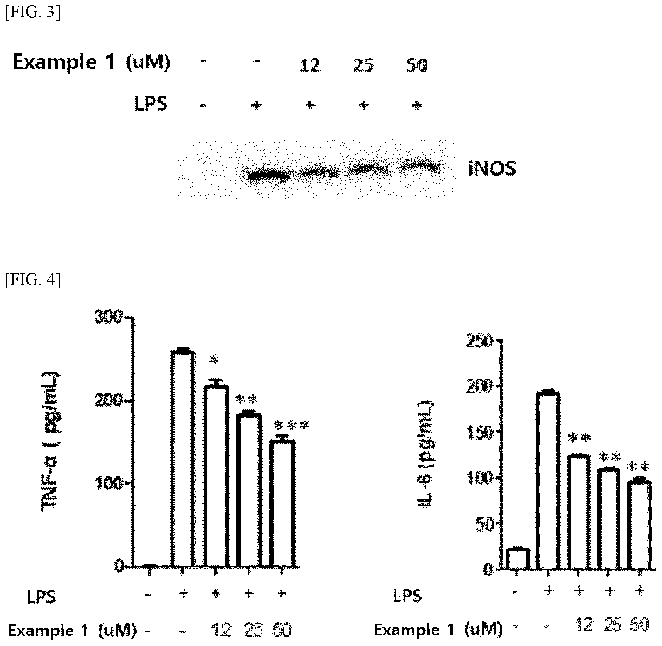

In order to confirm whether the NO production inhibitory effect of the compound of Example 1 is due to inhibition of iNOS protein expression, 1 mg/mL LPS and the compound of Example 1 at concentrations of 0, 12 µM, 25 µM, and 50 µM were added to Raw264.7 cells, and the effect on the expression of iNOS protein was evaluated by the method in <Experiment Method> 7 above. As a result, Raw264.7 cells to which LPS was not added did not express iNOS protein, and Raw264.7 cells to which 1 mg/mL LPS was added showed high iNOS protein expression. In addition, when treated with the compound of Example 1, it was confirmed that iNOS protein expression in LPS-stimulated Raw264.7 was significantly suppressed (FIG. 3).

### <Experimental Example 5> Analysis of inflammation-related factors production inhibitory effect

LPS, well known as an endotoxin present in the outer cell membrane of Gram-negative bacteria, stimulates macrophages or monocytes to promote the secretion of inflammatory cytokines such as tumor necrosis factor-α (TNF-α), interleukin-1β (IL-1β), and interleukin-6 (IL-6). Accordingly, in order to analyze the effect of the compound of Example 1 on inhibiting cytokine secretion, RAW264.7 cells were cultured for 24 hours by adding 1 mg/mL of LPS and the compound of Example 1 at concentrations of 0, 12 µM, 25 µM, and 50 µM, and the concentrations of IL-6 and TNFα in the culture supernatant were measured. As a result, the compound of Example 1 effectively inhibited the production of TNFα and IL-6 in LPS-stimulated Raw264.7 cells in a concentration-dependent manner, and statistical significance was confirmed compared to the vehicle group (FIG. 4).

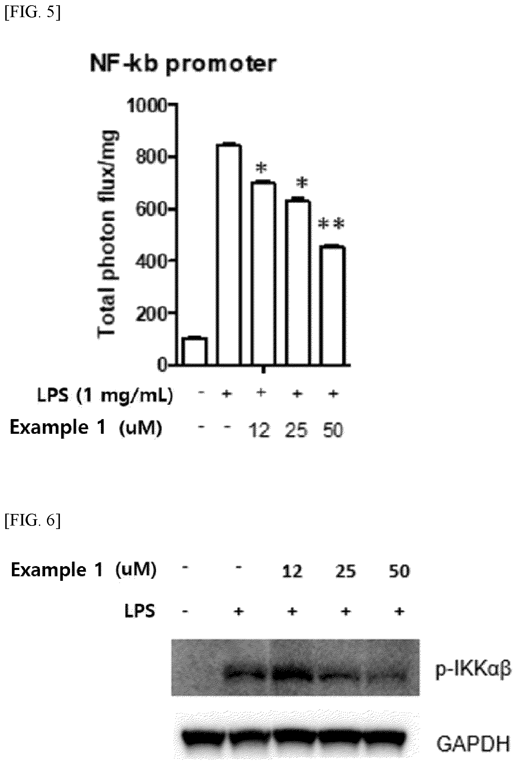

Nuclear factor-kappa B (NF-κB), which plays an important role in inflammatory responses, is a transcription factor that regulates the synthesis of various cytokines, chemokines, and growth factors. NFκB is composed of p50 and p65, enters the nucleus, acts as a transcription factor, and synthesizes iNOS, COX-2, and inflammation-related cytokines, and generally binds to the inhibitor NF-κBα (IκBα) in the cytoplasm, thereby inhibiting the action of NF-κB. In order to confirm whether NF-κb-related cell signaling induced in Raw264.7 cells was affected by the compound of Example 1, NF-κb promoter-luciferase analysis and pIKKαβ expression change analysis were performed in Raw264.7 cells using 1 mg/mL LPS and the compound of Example 1 at concentrations of 0, 12 µM, 25 µM, and 50 µM. As a result, when 1 mg/ml LPS was treated with Raw264.7/NF- 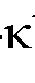 b-luciferase cells, strong luminescent signals occurred, but when treated with the compound of Example 1, the luminescent signals decreased in a concentration-dependent manner, and statistical significance was confirmed when compared to the vehicle group (FIG. 5). In addition, it was confirmed that pIKKαβ protein expression was effectively reduced by the compound of Example 1 (FIG. 6).

Through this, it can be seen that the compound of Example 1 regulates NF- 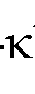 b cell signaling in macrophages as a PPARδ agonist and is effective in controlling inflammatory cytokines.

### <Experimental Example 6> Macrophage image analysis

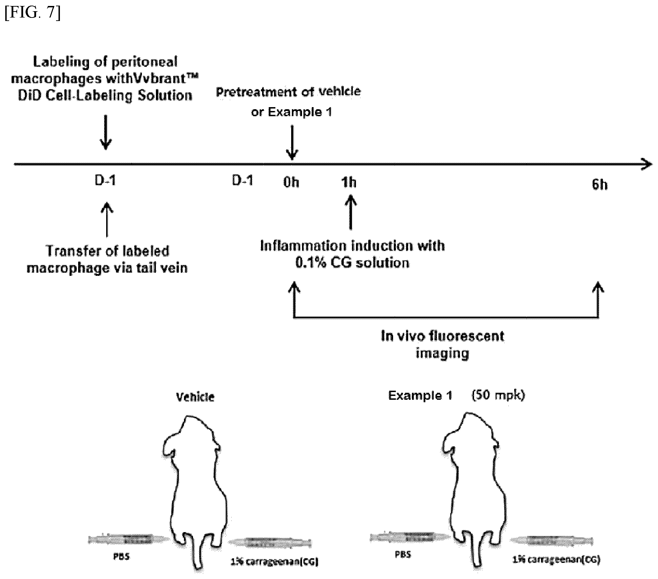

The inflammation model induced by λ-carrageenan in the mouse foot region is an acute arthritis model and is useful when checking the effect of suppressing edema due to osteoarthritis. In order to confirm the ability of the compound of Example 1 to suppress acute inflammation, an experiment was performed according to the macrophage tracking technique (FIG. 7) and <Experiment Method> 8 above.

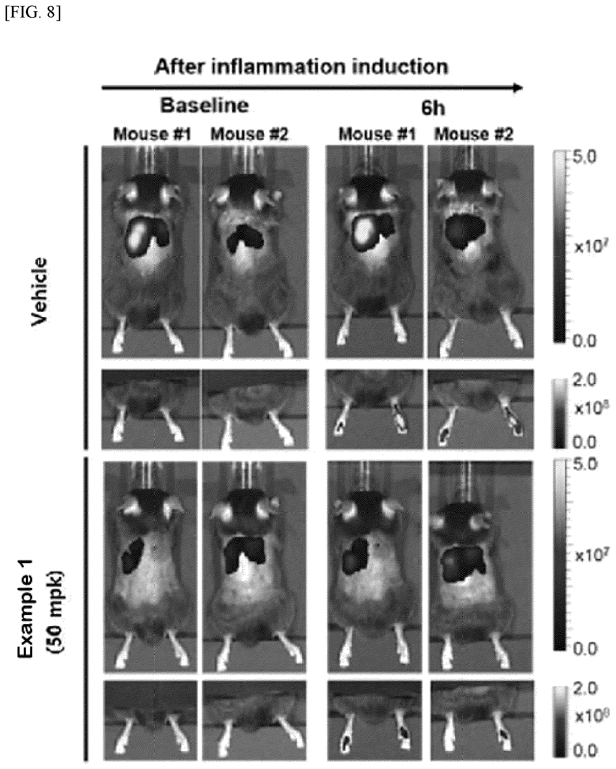

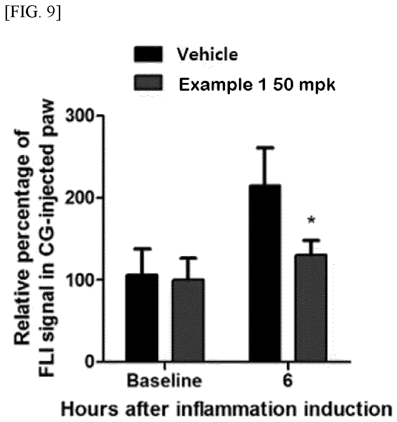

As a result, no fluorescent signal could be confirmed in the soles of all experimental groups injected with 0.1% CG before inducing inflammation. On the other hand, 6 hours after inducing inflammation, a strong fluorescent signal was confirmed in the sole of the foot injected with 0.1% CG in the vehicle group. Meanwhile, in the sole of the foot injected with 0.1% CG in the group treated with 50 mg/kg compound of Example 1, a lower fluorescenct signal was confirmed than that in the vehicle group, and statistical significance was also detected (FIGS. 8 and 9).

Through this, it could be confirmed that the compound of Example 1, which is a PPARδ agonist, has no cytotoxicity and is effective in suppressing the production of inflammatory cytokines and NO by regulating NF-xb cell signaling in macrophages. It was also confirmed to be effective in preventing macrophage infiltration into acute inflammatory areas.

Although the present disclosure has been described in detail through preferred embodiments and experimental examples above, the scope of the present disclosure is not limited to the specific embodiments and should be construed in accordance with the appended claims. In addition, those skilled in the art should understand that many modifications and variations are possible without departing from the scope of the present disclosure.

## Claims

1. A compound represented by the following Chemical Formula 1, a stereoisomer thereof, a hydrate thereof, or a salt thereof: 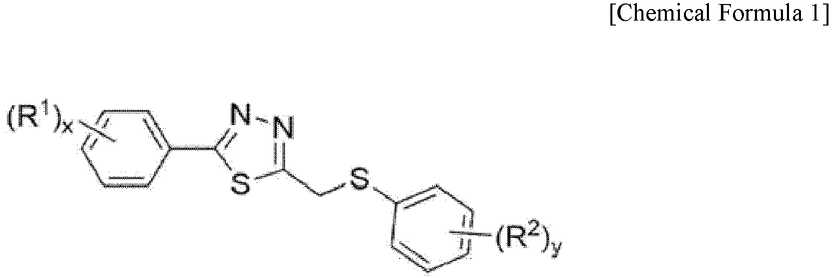
wherein, in the Chemical Formula 1,
R¹ is each independently -H, halogen, C₁₋₅alkyl, C₁₋₅haloalkyl, C₁₋₅alkoxy or C₁₋₅haloalkoxy;
R² is each independently -H, C₁₋₅alkyl or -O-R²¹,
wherein the R²¹ is cyano C₁₋₅alkyl, heteroaryl C₁₋₅alkyl of 5 to 8 atoms containing one or more heteroatoms selected from the group consisting of N, O and S, or 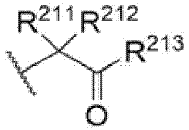 ,
wherein the R²¹¹ and R²¹² are each independently -H or C₁₋₅alkyl, the R²¹³ is -OH, - NH₂, -NHOR²¹⁴ or -NHCN, and
the R²¹⁴ is -H or C₁₋₅alkyl; and
x and y are each independently integers from 1 to 5.

2. The compound of claim 1, a stereoisomer thereof, a hydrate thereof, or a salt thereof, wherein the compound represented by the Chemical Formula 1 is a compound represented by the following Chemical Formula 2: 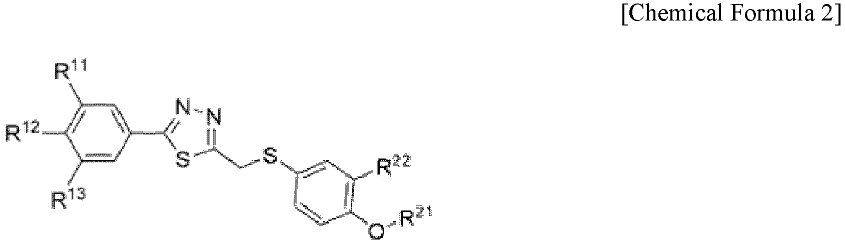
wherein, in the Chemical Formula 2,
R¹¹, R¹² and R¹³ are each independently -H, halogen, C₁₋₅alkyl, C₁₋₅haloalkyl or C₁₋₅haloalkoxy;
R²² is -H or C₁₋₅alkyl; and
R²¹ is cyano C₁₋₅alkyl, heteroaryl C₁₋₅alkyl of 5 to 8 atoms containing one or more heteroatoms selected from a group consisting of N, O and S, or 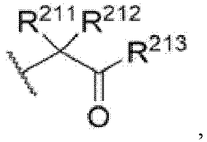
wherein the R²¹¹ and R²¹² are each independently -H or C₁₋₅alkyl, the R²¹³ is -OH, - NH₂, -NHOR²¹⁴ or -NHCN, and
the R²¹⁴ is -H or C₁₋₅alkyl.

3. The compound of claim 1, a stereoisomer thereof, a hydrate thereof, or a salt thereof, wherein the compound represented by the Chemical Formula 1 is a compound represented by the following Chemical Formula 3: 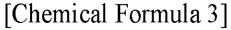 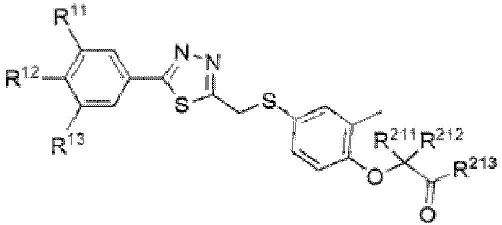
wherein, in the Chemical Formula 3,
R¹¹, R¹² and R¹³ are each independently -H, halogen, C₁₋₅alkyl, C₁₋₅haloalkyl or C₁₋₅haloalkoxy;
R²¹¹ and R²¹² are each independently -H or C₁₋₅alkyl; and
R²¹³ is -OH, -NH₂, -NHOR²¹⁴ or -NHCN, and the R²¹⁴ is -H or C₁₋₅alkyl.

4. The compound of claim 1, a stereoisomer thereof, a hydrate thereof, or a salt thereof, wherein the R²¹ is

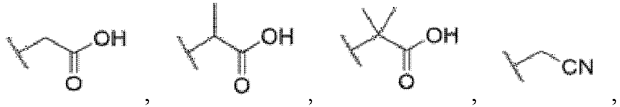

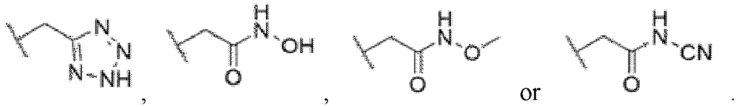

5. The compound of claim 1, a stereoisomer thereof, a hydrate thereof, or a salt thereof, wherein the R¹ is each independently -H, halogen, -CH₃, -CF₃ or -OCF₃.

6. The compound of claim 1, a stereoisomer thereof, a hydrate thereof, or a salt thereof, wherein the compound represented by Chemical Formula 1 is any one selected from the following group of compounds:
(1) 2-(2-methyl-4-(((5-(4-(trifluoromethyl)phenyl)-1,3,4-thiadiazol-2-yl)methyl)thio)phenoxy)acetic acid;
(2) 2-(2-methyl-4-(((5-(4-(trifluoromethyl)phenyl)-1,3,4-thiadiazol-2-yl)methyl)thio)phenoxy)propanoic acid;
(3) 2-methyl-2-(2-methyl-4-(((5-(4-(trifluoromethyl)phenyl)-1,3,4-thiadiazol-2-yl)methyl)thio)phenoxy)propanoic acid;
(4) 2-(2-methyl-4-(((5-(4-(trifluoromethyl)phenyl)-1,3,4-thiadiazol-2-yl)methyl)thio)phenoxy)acetonitrile;
(5) 2-(((4-((2H-tetrazol-5-yl)methoxy)-3-methylphenyl)thio)methyl)-5-(4-(trifluoromethyl)phenyl)-1,3,4-thiadiazole;
(6) N-hydroxy-2-(2-methyl-4-(((5-(4-(trifluoromethyl)phenyl)-1,3,4-thiadiazol-2-yl)methyl)thio)phenoxy)acetamide;
(7) N-methoxy-2-(2-methyl-4-(((5-(4-(trifluoromethyl)phenyl)-1,3,4-thiadiazol-2-yl)methyl)thio)phenoxy)acetamide;
(8) N-cyano-2-(2-methyl-4-(((5-(4-(trifluoromethyl)phenyl)-1,3,4-thiadiazol-2-yl)methyl)thio)phenoxy)acetamide;
(9) 2-(4-(((5-(4-fluorophenyl)-1,3,4-thiadiazol-2-yl)methyl)thio)-2-methylphenoxy)acetic acid;
(10) 2-(4-(((5-(4-fluorophenyl)-1,3,4-thiadiazol-2-yl)methyl)thio)-2-methylphenoxy)propanoic acid;
(11) 2-(4-(((5-(4-fluorophenyl)-1,3,4-thiadiazol-2-yl)methyl)thio)-2-methylphenoxy)-2-methylpropanoic acid;
(12) 2-(4-(((5-(3,4-difluorophenyl)-1,3,4-thiadiazol-2-yl)methyl)thio)-2-methylphenoxy)acetic acid;
(13) 2-(4-(((5-(3,4-difluorophenyl)-1,3,4-thiadiazol-2-yl)methyl)thio)-2-methylphenoxy)propanoic acid;
(14) 2-(4-(((5-(3,4-difluorophenyl)-1,3,4-thiadiazol-2-yl)methyl)thio)-2-methylphenoxy)-2-methylpropanoic acid;
(15) 2-(2-methyl-4-(((5-(3,4,5-trifluorophenyl)-1,3,4-thiadiazol-2-yl)methyl)thio)phenoxy)acetic acid;
(16) 2-(4-(((5-(3-chloro-4-fluorophenyl)-1,3,4-thiadiazol-2-yl)methyl)thio)-2-methylphenoxy)acetic acid;
(17) 2-(4-(((5-(3-chloro-4-fluorophenyl)-1,3,4-thiadiazol-2-yl)methyl)thio)-2-methylphenoxy)propanoic acid;
(18) 2-(4-(((5-(3-chloro-4-fluorophenyl)-1,3,4-thiadiazol-2-yl)methyl)thio)-2-methylphenoxy)-2-methylpropanoic acid;
(19) 2-(4-(((5-(3-fluoro-4-(trifluoromethyl)phenyl)-1,3,4-thiadiazol-2-yl)methyl)thio)-2-methylphenoxy)acetic acid;
(20) 2-(4-(((5-(3-fluoro-4-(trifluoromethyl)phenyl)-1,3,4-thiadiazol-2-yl)methyl)thio)-2-methylphenoxy)propanoic acid;
(21) 2-(4-(((5-(3-fluoro-4-(trifluoromethyl)phenyl)-1,3,4-thiadiazol-2-yl)methyl)thio)-2-methylphenoxy)-2-methylpropanoic acid;
(22) 2-(2-methyl-4-(((5-(p-tolyl)-1,3,4-thiadiazol-2-yl)methyl)thio)phenoxy)acetic acid;
(23) 2-(2-methyl-4-(((5-(p-tolyl)-1,3,4-thiadiazol-2-yl)methyl)thio)phenoxy)propanoic acid;
(24) 2-methyl-2-(2-methyl-4-(((5-(p-tolyl)-1,3,4-thiadiazol-2-yl)methyl)thio)phenoxy)propanoic acid;
(25) 2-(2-methyl-4-(((5-(4-(trifluoromethoxy)phenyl)-1,3,4-thiadiazol-2-yl)methyl)thio)phenoxy)acetic acid;
(26) 2-(2-methyl-4-(((5-(4-(trifluoromethoxy)phenyl)-1,3,4-thiadiazol-2-yl)methyl)thio)phenoxy)propanoic acid; and
(27) 2-methyl-2-(2-methyl-4-(((5-(4-(trifluoromethoxy)phenyl)-1,3,4-thiadiazol-2-yl)methyl)thio)phenoxy)propanoic acid.

7. A pharmaceutical composition for preventing or treating inflammatory disease, comprising a compound represented by the following Chemical Formula 1, a stereoisomer thereof, a hydrate thereof, or a pharmaceutically acceptable salt thereof as an active ingredient: 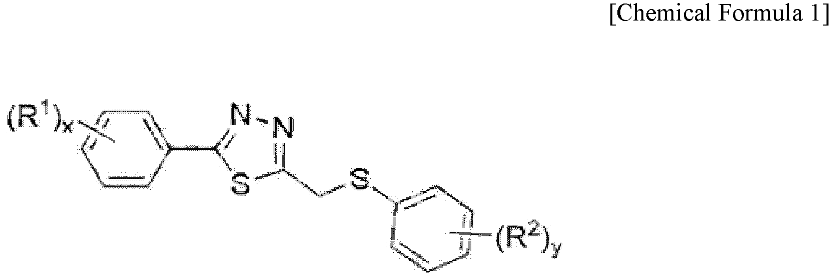
wherein, in the Chemical Formula 1,
R¹ is each independently -H, halogen, C₁₋₅alkyl, C₁₋₅haloalkyl, C₁₋₅alkoxy or C₁₋₅haloalkoxy;
R² is each independently -H, C₁₋₅alkyl or -O-R²¹,
wherein the R²¹ is cyano C₁₋₅alkyl, heteroaryl C₁₋₅alkyl of 5 to 8 atoms containing one or more heteroatoms selected from a group consisting of N, O and S, or 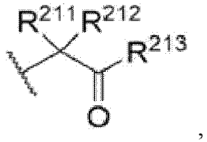
wherein the R²¹¹ and R²¹² are each independently -H or C₁₋₅alkyl, the R²¹³ is -OH, - NH₂, -NHOR²¹⁴ or -NHCN, and
the R²¹⁴ is -H or C₁₋₅alkyl; and
x and y are each independently integers from 1 to 5.

8. The pharmaceutical composition of claim 7, wherein the compound represented by the Chemical Formula 1 is a compound represented by the following Chemical Formula 2: 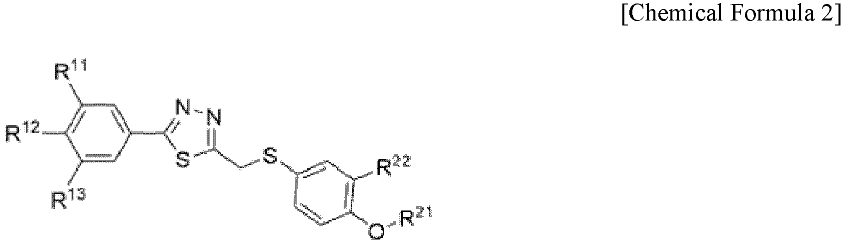
wherein, in the Chemical Formula 2,
R¹¹, R¹² and R¹³ are each independently -H, halogen, C₁₋₅alkyl, C₁₋₅haloalkyl or C₁₋₅haloalkoxy;
R²² is -H or C₁₋₅alkyl; and
R²¹ is cyano C₁₋₅alkyl, heteroaryl C₁₋₅alkyl of 5 to 8 atoms containing one or more heteroatoms selected from a group consisting of N, O and S, or 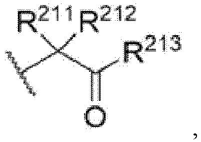
wherein the R²¹¹ and R²¹² are each independently -H or C₁₋₅alkyl, the R²¹³ is -OH, - NH₂, -NHOR²¹⁴ or -NHCN, and
the R²¹⁴ is -H or C₁₋₅alkyl.

9. The pharmaceutical composition of claim 7, wherein the compound represented by the Chemical Formula 1 is a compound represented by the following Chemical Formula 3: 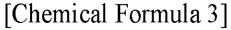 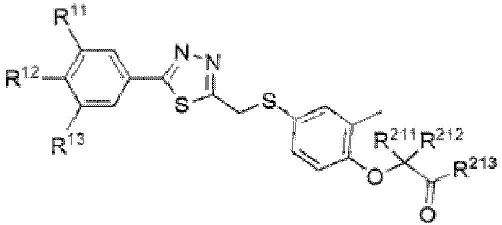
wherein, in the Chemical Formula 3,
R¹¹, R¹² and R¹³ are each independently -H, halogen, C₁₋₅alkyl, C₁₋₅haloalkyl or C₁₋₅haloalkoxy;
R²¹¹ and R²¹² are each independently -H or C₁₋₅alkyl; and
R²¹³ is -OH, -NH₂, -NHOR²¹⁴ or -NHCN, the R²¹⁴ is -H or C₁₋₅alkyl.

10. The pharmaceutical composition of claim 7, wherein the R²¹ is

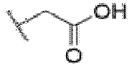

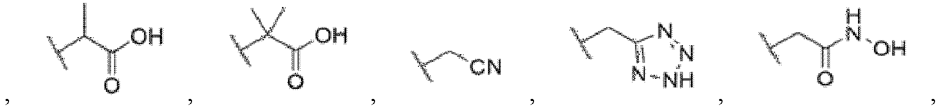

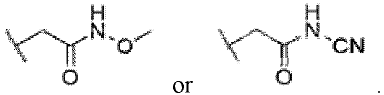

11. The pharmaceutical composition of claim 7, wherein the R¹ is each independently -H, halogen, -CH₃, -CF₃ or -OCF₃.

12. The pharmaceutical composition of claim 7, wherein the compound represented by Chemical Formula 1 is any one selected from the following group of compounds:
(1) 2-(2-methyl-4-(((5-(4-(trifluoromethyl)phenyl)-1,3,4-thiadiazol-2-yl)methyl)thio)phenoxy)acetic acid;
(2) 2-(2-methyl-4-(((5-(4-(trifluoromethyl)phenyl)-1,3,4-thiadiazol-2-yl)methyl)thio)phenoxy)propanoic acid;
(3) 2-methyl-2-(2-methyl-4-(((5-(4-(trifluoromethyl)phenyl)-1,3,4-thiadiazol-2-yl)methyl)thio)phenoxy)propanoic acid;
(4) 2-(2-methyl-4-(((5-(4-(trifluoromethyl)phenyl)-1,3,4-thiadiazol-2-yl)methyl)thio)phenoxy)acetonitrile;
(5) 2-(((4-((2H-tetrazol-5-yl)methoxy)-3-methylphenyl)thio)methyl)-5-(4-(trifluoromethyl)phenyl)-1,3,4-thiadiazole;
(6) N-hydroxy-2-(2-methyl-4-(((5-(4-(trifluoromethyl)phenyl)-1,3,4-thiadiazol-2-yl)methyl)thio)phenoxy)acetamide;
(7) N-methoxy-2-(2-methyl-4-(((5-(4-(trifluoromethyl)phenyl)-1,3,4-thiadiazol-2-yl)methyl)thio)phenoxy)acetamide;
(8) N-cyano-2-(2-methyl-4-(((5-(4-(trifluoromethyl)phenyl)-1,3,4-thiadiazol-2-yl)methyl)thio)phenoxy)acetamide;
(9) 2-(4-(((5-(4-fluorophenyl)-1,3,4-thiadiazol-2-yl)methyl)thio)-2-methylphenoxy)acetic acid;
(10) 2-(4-(((5-(4-fluorophenyl)-1,3,4-thiadiazol-2-yl)methyl)thio)-2-methylphenoxy)propanoic acid;
(11) 2-(4-(((5-(4-fluorophenyl)-1,3,4-thiadiazol-2-yl)methyl)thio)-2-methylphenoxy)-2-methylpropanoic acid;
(12) 2-(4-(((5-(3,4-difluorophenyl)-1,3,4-thiadiazol-2-yl)methyl)thio)-2-methylphenoxy)acetic acid;
(13) 2-(4-(((5-(3,4-difluorophenyl)-1,3,4-thiadiazol-2-yl)methyl)thio)-2-methylphenoxy)propanoic acid;
(14) 2-(4-(((5-(3,4-difluorophenyl)-1,3,4-thiadiazol-2-yl)methyl)thio)-2-methylphenoxy)-2-methylpropanoic acid;
(15) 2-(2-methyl-4-(((5-(3,4,5-trifluorophenyl)-1,3,4-thiadiazol-2-yl)methyl)thio)phenoxy)acetic acid;
(16) 2-(4-(((5-(3-chloro-4-fluorophenyl)-1,3,4-thiadiazol-2-yl)methyl)thio)-2-methylphenoxy)acetic acid;
(17) 2-(4-(((5-(3-chloro-4-fluorophenyl)-1,3,4-thiadiazol-2-yl)methyl)thio)-2-methylphenoxy)propanoic acid;
(18) 2-(4-(((5-(3-chloro-4-fluorophenyl)-1,3,4-thiadiazol-2-yl)methyl)thio)-2-methylphenoxy)-2-methylpropanoic acid;
(19) 2-(4-(((5-(3-fluoro-4-(trifluoromethyl)phenyl)-1,3,4-thiadiazol-2-yl)methyl)thio)-2-methylphenoxy)acetic acid;
(20) 2-(4-(((5-(3-fluoro-4-(trifluoromethyl)phenyl)-1,3,4-thiadiazol-2-yl)methyl)thio)-2-methylphenoxy)propanoic acid;
(21) 2-(4-(((5-(3-fluoro-4-(trifluoromethyl)phenyl)-1,3,4-thiadiazol-2-yl)methyl)thio)-2-methylphenoxy)-2-methylpropanoic acid;
(22) 2-(2-methyl-4-(((5-(p-tolyl)-1,3,4-thiadiazol-2-yl)methyl)thio)phenoxy)acetic acid;
(23) 2-(2-methyl-4-(((5-(p-tolyl)-1,3,4-thiadiazol-2-yl)methyl)thio)phenoxy)propanoic acid;
(24) 2-methyl-2-(2-methyl-4-(((5-(p-tolyl)-1,3,4-thiadiazol-2-yl)methyl)thio)phenoxy)propanoic acid;
(25) 2-(2-methyl-4-(((5-(4-(trifluoromethoxy)phenyl)-1,3,4-thiadiazol-2-yl)methyl)thio)phenoxy)acetic acid;
(26) 2-(2-methyl-4-(((5-(4-(trifluoromethoxy)phenyl)-1,3,4-thiadiazol-2-yl)methyl)thio)phenoxy)propanoic acid; and
(27) 2-methyl-2-(2-methyl-4-(((5-(4-(trifluoromethoxy)phenyl)-1,3,4-thiadiazol-2-yl)methyl)thio)phenoxy)propanoic acid.

13. The pharmaceutical composition of claim 7, wherein the inflammatory disease is inflammatory bowel disease, osteoarthritis, bronchitis, rheumatoid arthritis, degenerative arthritis, asthma, atopy, diabetes, myocardial infarction, Crohn's disease, or psoriasis.

14. A health functional food composition for preventing or ameliorating inflammatory disease, comprising a compound represented by the following Chemical Formula 1, a stereoisomer thereof, a hydrate thereof, or a salt thereof as an active ingredient: 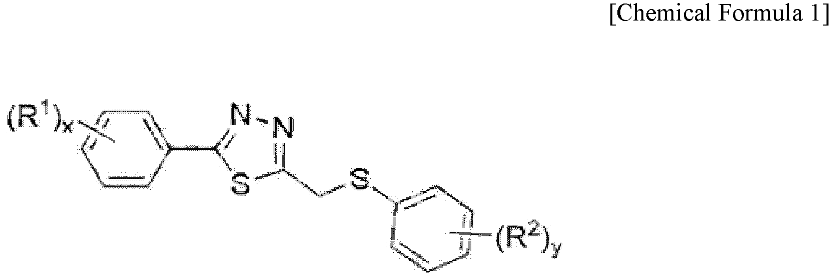
wherein, in the Chemical Formula 1,
R¹ is each independently -H, halogen, C₁₋₅alkyl, C₁₋₅haloalkyl, C₁₋₅alkoxy or C₁₋₅haloalkoxy;
R² is each independently -H, C₁₋₅alkyl or -O-R²¹,
wherein the R²¹ is cyano C₁₋₅alkyl, heteroaryl C₁₋₅alkyl of 5 to 8 atoms containing one or more heteroatoms selected from a group consisting of N, O and S, or 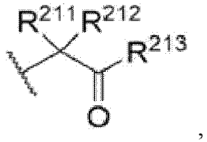
wherein the R²¹¹ and R²¹² are each independently -H or C₁₋₅alkyl, the R²¹³ is -OH, - NH₂, -NHOR²¹⁴ or -NHCN, and
the R²¹⁴ is -H or C₁₋₅alkyl; and
x and y are each independently integers from 1 to 5.
